# EUROPEAN PATENT SPECIFICATION

(11) **EP 0 376 041 B1**
(45) Date of publication and mention of the grant of the patent: **28.02.1996**
(21) Application number: 89122874.4
(22) Date of filing: 12.12.1989
(51) Int. Cl.: C07K 9/00

(54) **C63-Amide derivatives of 34-de(acetylglucosaminyl)-34-deoxy-teicoplanins**
C63-Amidderivate von 34-de(acetylglucosaminyl)-34-deoxy-teicoplaninen
Dérivés amides en C63 des 34-de(acétylglucosaminyl)-34-déoxyteicoplanines

(30) Priority: 27.12.1988 EP 88121708
(43) Date of publication of application: 04.07.1990
(73) Proprietor: GRUPPO LEPETIT S.p.A., I-20020 Lainate (MI) (IT)
(72) Inventor: Malabarba, Adriano, I-20082 Binasco (MI) (IT); Kettenring, Jürgen Kurt, I-21100 Varese (IT)
(74) Representative: Sgarbi, Renato

(56) References cited:
- EP-A- 0 218 099
- WO-A-87/03285
- WO-A-88/06600

## Description

### Description

This invention is directed to C63-amide derivatives of 34-de(acetylglucosaminyl)-34-deoxy-teicoplanins of the formula I 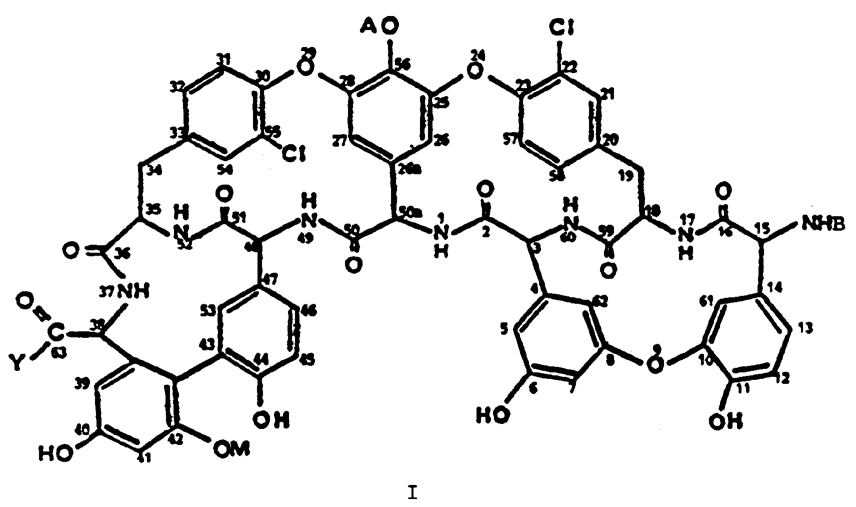 wherein:
A represents N[(C₉-C₁₂)aliphatic acyl]-beta-D-2-deoxy-2-aminoglucopyranosyl;
B is hydrogen or a protecting group of the amine function;
M represents alpha-D-mannopyranosyl;
Y represents a di- or poly-amine group of the formula 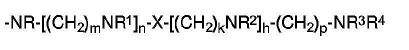 wherein:
R is hydrogen or linear or branched (C₁-C₈)alkyl;
R¹ is hydrogen or linear or branched (C₁-C₈)alkyl;
R² is hydrogen or linear or branched (C₁-C₈)alkyl;
R3 and R⁴ are each independently hydrogen, linear or branched (C₁-C₈)alkyl optionally bearing a NH₂, OH or SH substituent or taken together with the adjacent nitrogen atom, form a 5 to 7 membered saturated heterocyclic ring which may contain a further heteroatom selected from -S-, -O- and -NR⁵⁻ wherein R⁵ is hydrogen, (C₁-C₄)alkyl, phenyl, or phenyl-(C₁-C₄)alkyl
m, k and p each independently represent an integer from 2 to 8;
n and h each independently, represent an integer from 0 to 4;
X represents a single bond, or when n is 1, taken together with the adjacent group NR¹, it may represent a bifunctional radical of the formula: 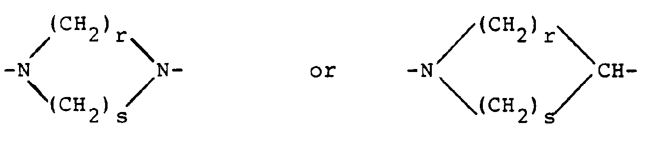 wherein r and s each independently represent an integer from 1 to 6 with the proviso that their sum is an integer from 3 to 8; and their addition salts with acids.

According to a preferred embodiment of this invention, the (C₉-C₁₂) aliphatic acyl radicals of the symbol A preferably are fully saturated or have one unsaturation. Most preferably, they are the following radicals: (Z)-4-decenoyl, 8-methylnonanoyl, decanoyl, 8-methyldecanoyl, 9-methyldecanoyl, 6-methyloctanoyl, nonanoyl, 10-methylundecanoyl and dodecanoyl.

The symbols R, R¹ and R², preferably represent hydrogen or linear or branched alkyl radicals of 1 to 4 carbon atoms.

The symbols R3 and R⁴ each independently preferably represents hydrogen, linear or branched alkyl radicals of 1 to 4 carbon atoms optionally bearing a NH₂, OH or SH substituent or R³ and R⁴ taken together with the adjacent nitrogen atom form a 5 to 7 membered saturated heterocyclic ring which may contain a further heteroatom selected from -S-, - 0- and -NR⁵-, the following heterocyclic rings being the most preferred ones: pyrrolidine, piperidine, oxazolidine, thiazolidine, isoxazolidine, isothiazolidine, morpholine, piperazine, thiomorpholine, hexahydroazepine, hexahydro-1,5-diazepine and hexahydro-1,4-diazepine; R⁵ preferably is hydrogen or Cₗ-C₄ alkyl.

The symbols m, k and p preferably represent integers from 2 to 6, most preferably, from 2 to 4.

The symbols n and h preferably represent 0, 1 or 2, most preferably 0 or 1.

The symbol X preferably represents a single bond or, when n is 1, taken together with the adjacent group NR¹ represents a bifunctional radical of the formula 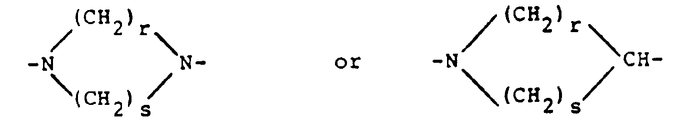 wherein r and s are both 2 or one is 1 and the other is 2 or 3.

According to the general definitions given above representative examples of the group: 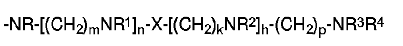 are the following:
-NH(CHV2NH2;
-NH(CH₂)₃N(CH₃)₂:
-NCH₃(CH₂)₃N(CH₃)₂;
-NC₂H₅(CH₂)₃N(n-C₄H₉)₂;
-NH(CH₂)₃NH(n-C₈H₁₇);
-NCH₃(CH₂)₃NHCH₃; 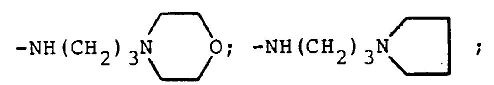
-NH(CH₂)₃NH(CH₂)₂OH; -NH(CH₂)₂NH(CH₂)₄SH;
-NCH₃(CH₂)₄-NC₂H₅(CH₂)₂NHC₂H₅;
-NH(CH₂)₄NH₂;
-NCH₃(CH₂)₆N(CH₃)₂;
-NC₂H₅(CH₂)₅NH₂;

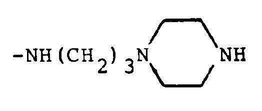

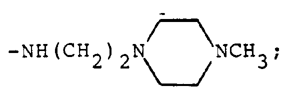

-NH(CH₂)₂NH(CH₂)₂NH₂:
-NH(CH₂)₃NH(CH₂)₃NH₂;
-NH(CH₂)₃N[(CH₂)₃NH₂]₂;
-NH(CH₂)₃N[(CH₂)₃OH]₂:
-NH(CH₂)₃NH(CH₂)₄NH₂;
-NH(CH₂)₄NH(CH₂)₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂)₃NH(CH₂)₄NH(CH₂)₃NH₂;

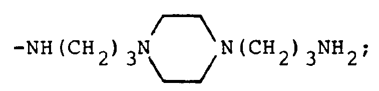

-NH(CH₂CH₂NH)₂CH₂CH₂NH₂;
-NH(CH₂CH₂CH₂NH)₃CH₂CH₂CH₂NH₂;
-NCH₃(CH₂)₂NH(CH₂)₃N(CH₃)₂;
-NCH₃(CH₂)₃NCH₃(CH₂)₃N(CH₃)₂;
-NCH₃(CH₂)₃NH(CH₂)₄N(n-C₄H₉)₂;
-NH(CH₂)₃NH(CH₂)₄NH(n-C₈H₁₇);
-NH(CH₂)₃NH(CH₂)₄NH(CH₂)₃N(n-C₄H₉)₂;
-NH(CH₂)₃NH(CH₂)₄NH(CH₂)₃NH(n-C₈H₁₇);
-NCH₃(CH₂)₃NCH₃(CH₂)₃NCH₃(CH₂)₃NHCH₃;
-NCH₃(CH₂)₃NCH₃(CH₂)₃NCH₃(CH₂)₃N(n-C₄H₉)₂;

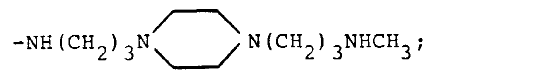

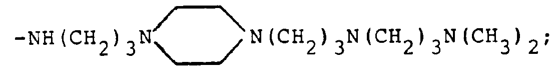

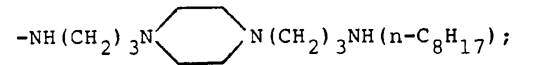

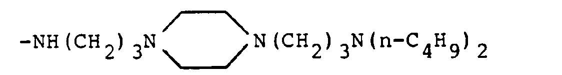

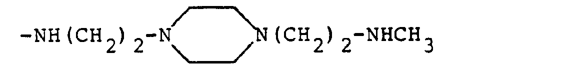

The compounds of this invention show antimicrobial activity, in particular, against gram-positive bacteria, including Group A Streptococci and some coagulase-negative Staphylococci.

Various C63-amide derivatives of teicoplanin complex, single components and the aglycone and pseudo aglycones thereof are described in European Patent Application Publication No. 218099 and International Patent Application Publication No. WO 88/06600.

The compounds of this invention are prepared by amidation of the corresponding 34-de(acetylglucosaminyl)-34-deoxy-teicoplanin derivatives of formula I wherein Y is OH, (i.e. the corresponding carboxy acids). These starting materials are specifically described in the European Patent Application Publication No. 290922 or can be prepared according to the procedure disclosed therein.

The above mentioned starting materials are prepared either from the teicoplanin A₂ complex (as resulting from fermentation operations) or from its five main components (see: U.S. Patent 4,542,018; A. Borghi et al., J. Antibiot. Vol. 37, 615-620, 1984; C.J. Barna et al. J. Am. Chem. Soc. 1984, 106, 4895-4902) by elimination of the acetylglucosaminyl rest at the position 34.

As it is known in the art, the above mentioned five main components of teicoplanin A₂ complex are characterized by the fact that the aliphatic acyl moiety of the beta-D-2-deoxy-2-aminoglucopyranosyl rest is a (C₁₀-C₁₁) aliphatic acyl, namely:(Z)-4-decenoyl, 8-methylnonanoyl, decanoyl, 8-methyldecanoyl or 9-methyldecanoyl. Accordingly, the resulting substances used as starting material for the manufacture of the compounds of this invention can be either individual products or mixtures of one or more products. Since said starting materials for the preparation of the compounds of this invention can be used in both said forms, the resulting end products may, in turn, be individual compounds or mixtures of two or more compounds of the above formula I. These mixtures of compounds are also part of the invention and may be used as such for their biological applications and uses or may be eventually separated in their individual components by known procedures described in the art. Examples of separation procedures suitable for the purpose of obtaining individual components from end products mixtures of teicoplanin amide derivatives are those described in the following documents: European Patent Application Publication No. 218099 and International Patent Application Publication No. WO 88/06600.

Other starting materials for the preparation of the compounds of this invention can be obtained by applying the process of the European Patent Application Publication No. 290922 to teicoplanin compounds such as those described as compound B (identified also as "RS-4" in the papers mentioned below) and compound A (identified also as "RS-3" in the papers mentioned below) in the European Patent Application Publication No. 306645, and those identified as teicoplanin compounds RS-1 and RS-2 in the paper given by M. Zanol et al. at the 17th International Symposium on Chromatography, Vienna, September 25-30, 1988 (see also A. Borghi et al. The Journal of Antibiotics, Vol. 42, No. 3, 361-366, 1989). Said teicoplanin compounds are characterized by the fact that the aliphatic acyl moieties of the beta-D-2-deoxy-2-amino-glucopyranosyl rest are respectively: nonanoyl, 6-methyloctanoyl, 10-methylundecanoyl and dodecanoyl.

The amidation procedures described in the above mentioned European Patent Application Publication No. 218099 and International Patent Application Publication No. WO 88/06600 can be used also for the preparation of the compounds of this invention. Said procedures involve condensing the carboxy acid starting materials mentioned above with an excess of the appropriate amine of the formula II: 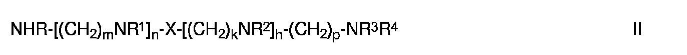 wherein R, R1 R2, R3, R4, X, m, n, h, k and p have the same meanings as above,
in an inert organic solvent in the presence of a condensing agent selected from (C₁-C₄)alkyl, phenyl or heterocyclyl phosphorazidates at a temperature between 0°C and 20°C. If the amine reactant contains other functions which are not inert under the selected reaction conditions, said functions are suitably protected by means of per se known protecting groups.

According to a preferred embodiment of this invention, the compounds of formula I wherein Y is a di-or poly-amine group as defined above are prepared by reacting an "activated ester" of the carboxylic acid of the same formula I, wherein Y is OH and the N¹⁵ -amino function is preferably protected, with the appropriate amine II.

The N¹⁵⁻amino function can be protected by methods known per se in the art such as those described in reference books like T.W. Greene, "Protective Groups in Organic Synthesis", John Wiley and Sons, New York, 1981, and M. Mc. Omie "Protecting Groups in Organic Chemistry", Plenum Press, New York, 1973. These protecting groups must be stable at the conditions of the reaction process, must not unfavorably interfere with the amidation reaction, and must be easily cleavable and removable from the reaction medium at the end of the reaction without altering the newly formed amide bond.

Representative examples of N-protecting groups which may be advantageously used in the process of the invention for protecting the N¹⁵ primary amino function of the teicoplanin starting material and, when appropriate, the NR³R⁴ moiety of the amine II reactant, are carbamate forming reagents characterized by the following oxycarbonyl groups: 1,1-dimethylpropynyloxycarbonyl, t-butyloxycarbonyl, vinyloxycarbonyl, aryloxycarbonyl, cinnamyloxycarbonyl, benzyloxycarbonyl, p-nitrobenzyloxycarbonyl, 3,4-dimethoxy-6-nitrobenzyloxycarbonyl, 2,4-dichlorobenzyloxycarbonyl, 5-ben- zisoxazolylmethyloxycarbonyl, 9-anthranylmethyloxycarbonyl, diphenylmethyloxycarbonyl, isonicotinyloxycarbonyl, S-benzyloxycarbonyl, 2,2,2-trichloroethoxycarbonyl, 2,2,2-trichloro-t-butoxycarbonyl, and the like. Other suitable N-protecting agents are aldehydes or ketones, or derivatives thereof which are capable of forming Schiff bases with the amino group to be protected.

Preferred examples of such Schiff base forming agents are benzaldehydes and particularly preferred is 2-hydroxybenzaldehyde (salicylaldehyde). A convenient means of protection in the case the amine 11 reactant has R different from hydrogen and contains a primary amino function (e.g. R3 and R⁴ both represent hydrogen) is, in some instances, the formation of a benzyliden derivative which may be prepared by reacting the amine with benzaldehyde in a lower alkanol, such as ethanol, preferably at room temperature. After the reaction with the selected teicoplanin starting material has been completed, the benzylidene protecting group may be removed as known in the art, e.g. by catalytic hydrogenation, using, for instance, Palladium on carbon as the catalyst.

However, in all cases where catalytic hydrogenation is applied, attention should be paid to the presence of groups which may be modified by catalytic hydrogenation. A typical consequence of the catalytic hydrogenation of an amino- protected derivative of formula I wherein A represents a group as above defined whose acyl portion is (Z)-4-decenoyl (or a mixture containing it) is that, at least partially, the decenoyl compound is transformed into the corresponding decanoyl compound. Therefore, when the removal of the protecting group is carried out through catalytic hydrogenation and the 34-de(acetylglucosaminyl)-34-deoxy-teicoplanin starting material is (or contains) a derivative of component 1 of teicoplanin A₂ complex (whose acyl portion is (Z)-4-decenoyl) the final amide product, in most cases, does not contain the corresponding derivative but, rather, a proportionally larger amount of the derivative of the component 3 whose acyl rest is decanoyl.

If a final compound containing the amide derivative of teicoplanin A₂ complex component 1 is desired, the N-protecting group must be selected among those which can be removed under conditions which do not imply hydrogenation of the acyl portion or hydrolysis of the sugar moieties of the teicoplanin substrate. For example, an N-protecting group which is removable under mild conditions is selected from beta-halo-alkoxycarbonyl groups, such as 2,2,2-trichloro-tert-butoxycarbonyl, which can be removed according to the procedures described by H. Eckert et al., in Angew. Chem. Int. Ed. Engl. 17, No.5, 361-362 (1978).

As it is appreciated by the skilled technician, the ultimate choice of the specific protecting group depends on the characteristics of the particular amide derivative which is desired. In fact, this amide function of the final compound should be stable at the conditions of removal of the protecting group(s).

Since the conditions of removal of the different protecting groups are known, the skilled technician is capable of selecting the proper protecting group.

In some cases, when the amine II contains two primary amino groups (e.g. R, R³ and R⁴ all representing hydrogen) it may be convenient to obtain a mixture of two reaction products resulting from the formation of the amidic bond with each of the two primary aminic functions and to separate them by common procedures such as flash column chromathography, reverse-phase column chromathography or preparative HPLC.

The formation of "activated esters" is described in general terms in Fieser and Fieser, "Reagents for Organic Synthesis", John Wiley and Sons Inc. 1967 pages 129-130.

Examples of said activated ester forming reagents that can be conveniently used in the process of the invention are those described by R. Schwyzer et al. in Helv. Chim. Acta, 1955, 38, 69-70 and encompass:
CICH₂CN, BrCH₂COOC₂H₅, BrCH(COOC_{Z}H_{S})₂, CICH₂COCH₃, 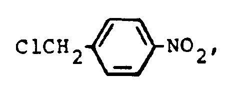
CICH₂CH₂N(C₂H₅)₂

A preferred reagent of this type is chloroacetonitrile. In this case, chloroacetonitrile itself or dimethylformamide (DMF) can be used as preferred solvents.

Generally, inert organic solvents useful for the formation of "activated esters" are those organic aprotic solvents which do not unfavorably interfere with the reaction course and are capable of, at least partially, solubilizing the carboxy acid starting material.

Examples of said inert organic solvents are organic amides, alkyl ethers, ethers of glycols and polyols, phosphoramides, sulfoxides and aromatic compounds. Preferred examples of inert organic solvents are: dimethylformamide, dimethoxyethane, hexamethylphosphoramide, dimethylsulfoxide, benzene, toluene and mixtures thereof.

More preferably, the solvent is selected from acetonitrile, dimethylsulfoxide, dimethylformamide. The formation of the activated ester is generally conducted in the presence of a base which does not interfere with the reaction course such as a tri-alkylamine like triethylamine, sodium or potassium carbonate or bicarbonate. Generally, the base is employed in a 2 to 6 molar proportion to the teicoplanin carboxy acid starting material and, preferably, it is used in an about three-fold molar excess. A preferred base is triethylamine.

The "activated ester" forming reagent is used in a large excess over the teicoplanin carboxy acid starting material. It is in general used in a 5 to 35 molar proportion and, preferably, it is used in an about 20 to 30 times molar excess. The reaction temperature is between 10°C and 60°C and preferably between 15°C and 30°C. As usual, the reaction time depends on the other specific reaction parameters and may be generally between 3 and 48 hours.

In this case, the reaction course may be followed by HPLC or TLC to determine when the reaction may be considered as completed and the procedures to recover the desired intermediate can be started. The "activated ester" intermediate can be directly used in the same reaction medium where it is prepared, however, in general, it is isolated by precipitation with non-solvents or by extraction with solvents and it is used as such, without further purification, in the next reaction step. If desired, however, it may be purified by column chromatography such as flash column chromatography or reverse-phase column chromatography.

The obtained "activated ester" intermediate is then reacted with a molar excess of the amine derivative of formula II 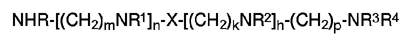 in the presence of an organic polar solvent at a temperature between 5°C and 60°C, preferably between 10°C and 30°C.

The organic polar solvent can be in this case a polar protic or aprotic solvent.

Preferred examples of organic polar protic solvents are lower(CᵣC₄) alkanols such as, ethanol, n-propanol, isopropanol, n-butanol and the like, or mixtures thereof, preferably used in the dry form.

Preferred examples of organic polar aprotic solvent are N,N-dimethylformamide (DMF), hexamethylphosphoramide (HMPA), or mixtures thereof, 1,3-dimethyi-3,4,5,6-tetrahydro-2(1 H)pyrimidone (DMPU), dimethylsulfoxide or dimethoxyethane.

The reaction of the "activated ester" with the selected amine can be carried out at a temperature between 5°C and 60°C but the preferred temperature is generally comprised between 10°C and 30°C, most preferably between 20°C and 25°C, while a preferred molar proportion between the "activated ester" intermediate and the amine II as above defined is from 1:5 to 1:30, and more preferably from 1:10 to 1:20. The reaction course may be monitored as usual by TLC or HPLC.

The amide derivative obtained from the amidation reaction is recovered from the reaction solution according to common procedures, for instance, by evaporation of the solvent or by addition of a non-solvent. The removal of the amino-protecting group is usually carried out on the crude product isolated from the amidation reaction.

Examples of procedures for the removal of said protecting groups from teicoplanin derivatives are described for instance in International Patent Application Publication No. WO 88106600.

If catalytic hydrogenation procedures are used, the reaction is usually carried out in the presence of a diluted aqueous strong acid, preferably a mineral acid, in an organic solvent miscible with said diluted aqueous strong acid. The filtrate from the reaction is then worked for the recovery of either the mineral acid addition salt of the amide of formula I or the corresponding free base. Analogous procedures are followed when the amino-protecting group is a group which can be removed by treating with diluted mineral acids (e.g. Schiff base or a C₁-C₄ alkoxy carbonyl group) under conditions which do not cause the splitting of the sugar moieties (e.g. low temperatures, short reaction time).

For the isolation of the acid addition salt, the reaction solution resulting from the splitting of the amino-protecting group is generally brought to a pH value between 6 and 7 by addition of an aqueous base, e.g. aqueous sodium hydroxide, and, after evaporation of the solvent under reduced pressure, the resulting solid is separated in the form of an addition salt with the strong acid which has been added during the de-protection step. Such product may be further purified by common techniques e.g. column chromatography, precipitation from solutions by addition of non-solvents, preparative HPLC and similar. The acid addition salt may be converted to the corresponding free base of formula I by suspending or dissolving the acid addition salt in an aqueous solvent which is then brought to an appropriate pH value whereby the free base form is restored. The product is then recovered, for instance, by extraction with an organic solvent or is transformed into another acid addition salt by adding the selected acid and working up as above.

Sometimes, after the above operation, it may be necessary to submit the recovered product to a common desalting procedure.

For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water or acetonitrile/aqueous acetic acid from 5% to about 100% acetonitrile and then recovered by evaporation of the solvent or by lyophilization.

When a compound of formula I is obtained in the free base form, it can be transformed into the corresponding acid addition salt by suspending or dissolving the free base form in an aqueous solvent and adding a slight molar excess of the selected acid. The resulting solution or suspension is then lyophilized to recover the desired acid addition salt. Instead of lyophilizing, in some instances, it is possible to recover the final salt through precipitation by addition of a non-solvent mixable with water.

In case the final salt is unsoluble in an organic solvent where the free base form is soluble it may be recovered by filtration from the organic solution of the non-salt form after addition of the stoichiometric amount or a slight molar excess of the selected acid.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, camphoric, glutaric, glycolic, phthalic, tartaric, lauric, stearic, salicylic, methanesulfonic, benzenesulfonic, sorbic, picric, benzoic, cinnamic and the like acids.

Preferred addition salts of the compounds of this invention are the pharmaceutically acceptable acid addition salts.

With the term "pharmaceutically acceptable acid addition salts" are intended those salts with acids which from biological, manufacturing and formulation standpoint are compatible with the pharmaceutical practice.

Examples of acids suitable for the "pharmaceutically acceptable acid addition salts" include those listed above.

A characteristic of the compounds of this invention which further differentiates them from the corresponding starting compound is the configuration of the amidic bond at the 51, 52 position which is "cis", while the configuration of the same bond in the starting teicoplanin carboxylic acid is "trans". This implies that the conformation of the teicoplanin core of the new compounds is remarkably modified with respect to that of the corresponding starting materials.

The compounds of the present invention in the form of both the free bases and their acid addition salts are useful as antibacterial agents, mainly active against gram-positive bacteria. More particularly, they are useful in the treatment of infections caused by Group A Streptococci (e.g. Streptococcus pyogenes). In fact, at present, they are the most active derivatives among teicoplanin antibiotics against the microorganisms of this genus. They are also more active than teicoplanin against coagulase-negative Staphylococci (e.g. Staphylococcus epidermidis and Staphylococcus haemo-Ivticus), in particular, Staphylococcus haemolyticus.

The antibacterial activity of the compounds of the invention is determined in vitro by means of standard agar-dilution tests in microtiter. Isosensitest broth (Oxoid) and Todd-Hewitt broth (Difco) are used for growing Staphylococci and Streptococci, respectively. Broth cultures are diluted so that the final inoculum is about 10⁴ forming units/ml (CFU/ml). Minimal inhibitory concentration (MIC) is considered as the lowest concentration which shows no visible growth after 18-24 h incubation at 37°C.

The results of the antibacterial testing of representative compounds of formula I are summarized in Table I.

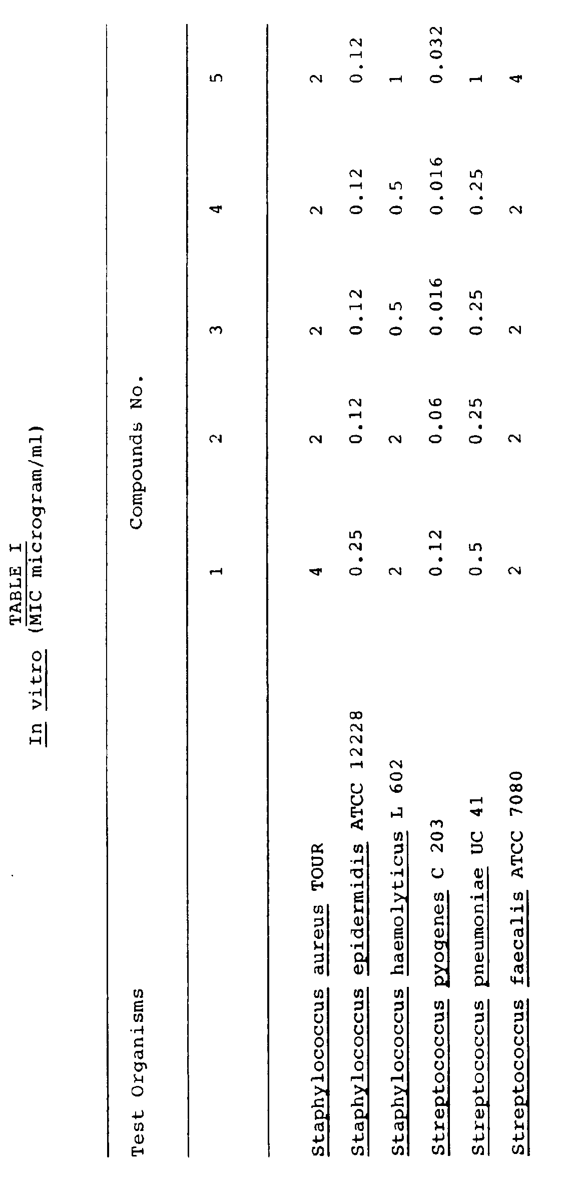

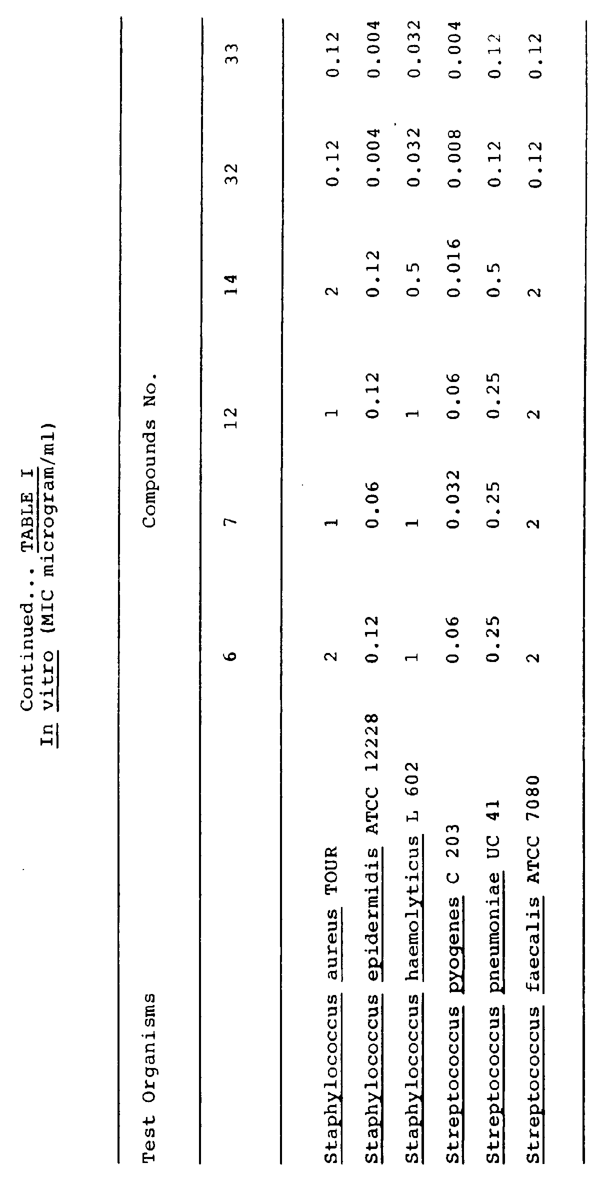

The activity of the compounds of this invention against Streptococcus pyogenes is, in some cases, higher than that of teicoplanin and the most active compounds of European Patent Application Publication No. 290922, European Patent Application Publication No. 218099 and International Patent Application Publication No. WO 88/06600 whose MIC (microgram/ml) against the same microorganism is never lower than 0.06.

For the most useful applications of the biological activity of the compounds of this invention, is of particular interest their selectivity against Streptococcus p^{y}o^{g}enes which is indicated by the comparison of the MIC values against said microorganism with the MIC values against the other test organisms reported in Table I above, in particular, Stachvlo- coccus aureus.

The activity against several clinical isolates of Streptococcus pyogenes of compounds 3, 5 and 32 is shown in Table I

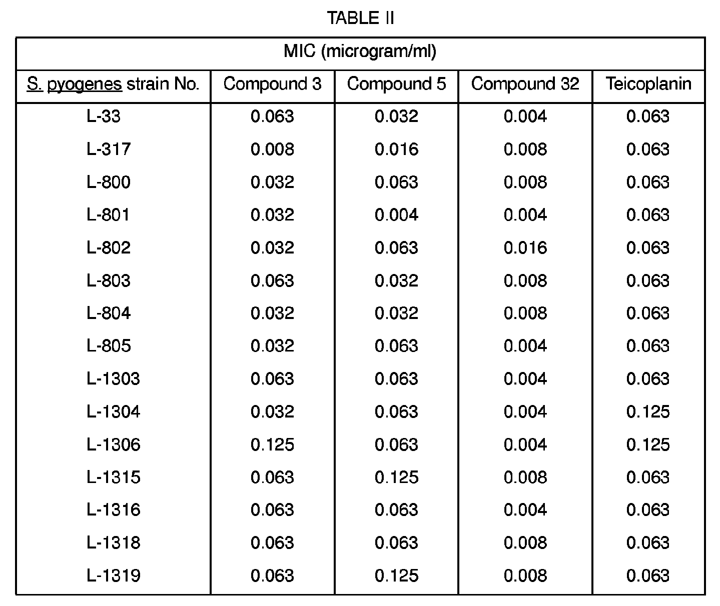

The compounds of this invention show considerably lower activity against bacteria other than Streptococci of Group A and coagulase negative Staphylococci and therefore they can be regarded as antibiotics showing a very narrow and selective spectrum of activity particularly useful for the specific target of combatting Streptococcal infections, with lower probability to select resistant strains of the other genera.

Streptococcal infections are usually responsible for severe pathological complications such as rheumatic fever, nephritis, endocarditis, erysipelas and the like.

The production of antibiotics with very narrow specific spectra is considered as an important need for the development of chemotherapy. See W. Brumfitt et al. in Postgraduate Medical Journal, Vol. 64 (1988) No. 753 pag. 552-558.

In view of the above reported antimicrobial activity, the compounds of the present invention can be employed as the active ingredient of antimicrobial preparations used in human and veterinary medicine for the prevention and treatment of infections diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms to be applied to the skin, the mucous membranes of the nose and throat or bronchial tissues and may conveniently take the form of creams, ointments, liquid sprays or inhalants, lozenges, or throat paints.

For medication of the eyes or ears, the preparation may be presented in liquid or semi-liquid form formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compounds of the invention are generally effective at a dosage comprised between about 0.3 and about 30 mg of active ingredient per kg of body weight, preferably divided in 2 to 4 administrations per day. Particularly desirable compositions are those prepared in the form of dosage units containing from about 20 to about 600 mg per unit.

### EXAMPLES

### GENERAL PROCEDURES

In the following examples the starting material may be the 34-de(acetylglucosaminyl)-34-deoxy teicoplanin A₂ complex (i.e. a mixture of compounds obtained from teicoplanin A₂ complex according to the procedure of European Patent Application Publication No. 290922, a single component thereof or any mixture of two or more of said components).

The typical complex mixture essentially consists of five components corresponding to formula I above wherein the aliphatic acyl moieties of the beta-D-2-deoxy-2-aminoglycopyranosyl radical represented by the symbol A are respectively:
(Z)-4-decenoyl (AC,), 8-methylnonanoyl (AC₂), decanoyl (AC₃), 8-methyldecanoyl (AC4) and 9-methyldecanoyl (AC₅), B is hydrogen, M is alpha-D-mannopyranosyl and Y is OH. This mixture is identified by the acronym TGAC₁₋₅. When one of the single components of said mixture is employed as the starting material it is identified as follows: TGAC₁, TGAC₂, TGAC₃, TGAC₄ or TGACₛ, depending on the specific aliphatic acyl rest of the above mentioned aminoglucop- yranosyl radical.

When a mixture of components is used it is indicated according to the same system as for the complex. For instance, the acronym TGAC₂₋₅ indicates the mixture of the components 2 to 5 wherein component 1 is no longer present. This mixture is currently obtained from teicoplanin A₂ complex according to the procedure of European Patent Application Publication No. 290922 when catalytic hydrogenation is applied which saturates the double bond of component 1 transforming it into component 3. The acronym TGAC₂,₃ indicates a mixture of the components 2 and 3 and the acronym TGAC₄,₅ indicates a mixture of the components 4 and 5.

The resulting end products in the following table III are identified by reference to formula I above with the indication for the symbol A of the particular aliphatic acyl substituent of the beta-D-2-deoxy-2-aminoglucopyianosyl radical (A/AC) by using the conventional terms AC₁, AC₂, AC₃, AC4, ACₛ as explained above. When a mixture of two or more components is obtained, this is shown through the same formal description as above.

HPLC Analysis is carried out with a Varian mod. 5000 LC pump equipped with a 20 microliter loop injector Rheodyne mod. 7125 and a UV detector at 254 nm. Columns: pre-column (1.9 cm) Hibar LiChro Cart 25-4 (Merck) pre-packed with Lichrosorb RP-8 (20-30 micrometer) followed by a column Hibar RT 250-4 (Merck) pre-packed with LiChrosorb RP-8 (10 micrometer). Eluents: A, 0.2% aq. HCOONH₄; B, CH₃CN. Flow rate: 2 mUmin. Iniection: 20 microliter. Elution: linear gradient from 20 to 60% of B in A in 30 min. The retention times of some representative compounds are reported in TABLE IIIb.

Acid-Base Titrations: The products are dissolved in MCS (methylcellosolve):H₂O 4:1 (v/v), then an excess of 0.01 M HCI in the same solvent mixture is added and the resulting solutions are titrated with 0.01 N NaOH. Equivalent weights of some representative compounds are reported in TABLE Ilia.

¹ H-NMR spectra at 500 MHz are recorded in the temperature range from 20°C to 30°C on a Bruker AM 500 spectrometer in DMSO=d₆ with tetramethylsilane (TMS) as the internal reference (delta = 0.00 ppm). Table Illc reports the most significant chemical shifts (delta, ppm) of some representative compounds.

### METHOD OF PREPARATION

a) A solution of 4 g (about 2.5 mmol) of a TGAC (the complex, a single component thereof, or a mixture of two or more of the single components) and 0.36 mL (about 2.6 mmol) of triethylamine (TEA) in 20 mL of DMF is stirred at room temperature for 30 min., while adding 0.4 mL (about 2.8 mmol) of benzyl chloroformate. Then, additional 0.4 mL (about 3.3 mmol) of TEA and 4 mL (about 65 mmol) of chloroacetonitrile are added and stirring is continued at room temperature for 20 h. The reaction mixture is poured into 300 mL of ethyl acetate, the precipitated solid is collected by filtration, and washed with 100 mL of ethyl ether, yielding (after drying in vacuo at room temperature overnight) 4.3 g of crude cyanomethyl ester of N¹⁵ -carbobenzyloxy TGAC.
b) To a stirred solution of the above product in 30 mL of DMF, 35 mmol of the proper reactant amine is added, and the resulting solution is stirred at room temperature overnight. Then, 25 mL of absolute ethanol is added, followed by 250 mL of ethyl acetate. The precipitated solid is collected by filtration, washed with 100 mL of ethyl ether, and dried in vacuo at room temperature for 4 hours, yielding 4.1 g of crude N¹⁵-carbobenzyloxy compound of formula I, which
c) is dissolved in 350 mL of a mixture methanol:0.01 N HCI 7:3 (v/v). The resulting solution is adjusted at pH 3.0 with 1 N HCI and hydrogenated at 1 atm and room temperature, in the presence of 4 g of 5% Pd/C, while absorbing 120 mL of hydrogen gas within 2 hours. The catalyst is filtered off and the clear filtrate is adjusted at pH 6.5 with 1 N NaOH. After adding 300 mL of n-butanol and 15 g of silanized Silica-gel (0.06-0.2 mm, Merck), solvents are evaporated at 40°C under reduced pressure. The solid residue is suspended in 200 mL of water and the resulting suspension is loaded at the top of a column of 400 g of the same silanized Silica-gel in water. The column is developed with a linear gradient from 10% to 80% of acetonitrile in 0.1 N acetic acid in 20 hours at the flow rate of about 250 mUh, while collecting 25 mL fractions, which are checked by HPLC. Those fractions containing pure compounds of the title are pooled, and the resulting solution is adjusted at pH 8.5 with 1 N NaOH, and then it is concentrated at 40°C under reduced pressure to a small volume (about 50 mL). The solid which separates is collected by centrifugation, washed with 10 mL of water, then with 250 mL of ethyl ether. After drying at room temperature in vacuo overnight, the compound of the formula I is obtained, as the free base.

For the manufacture of the invention compounds where the AC₁ moiety is still present, the step a) is modified by reacting the TGAC₁₋₅ complex or the TGAC₁, or a mixture of two or more components containing it, with 2,2,2-trichloro-t-butoxy-chloroformate according to the same procedure as above and the first portion of step c) is replaced by contacting the resulting C63-amide N¹⁵⁻protected amine with zinc in acetic acid according to the procedure described by H. Eckert et al. in Angew. Chem. Int. Ed. Engl. 17, No.5, 361-362 (1978). The purification is carried out in the same way as described in the second part of step c).

By using the appropriate reagents TGAC and a amine of formula: 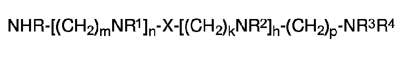 under the conditions described above, the compounds represented in Table III are obtained.

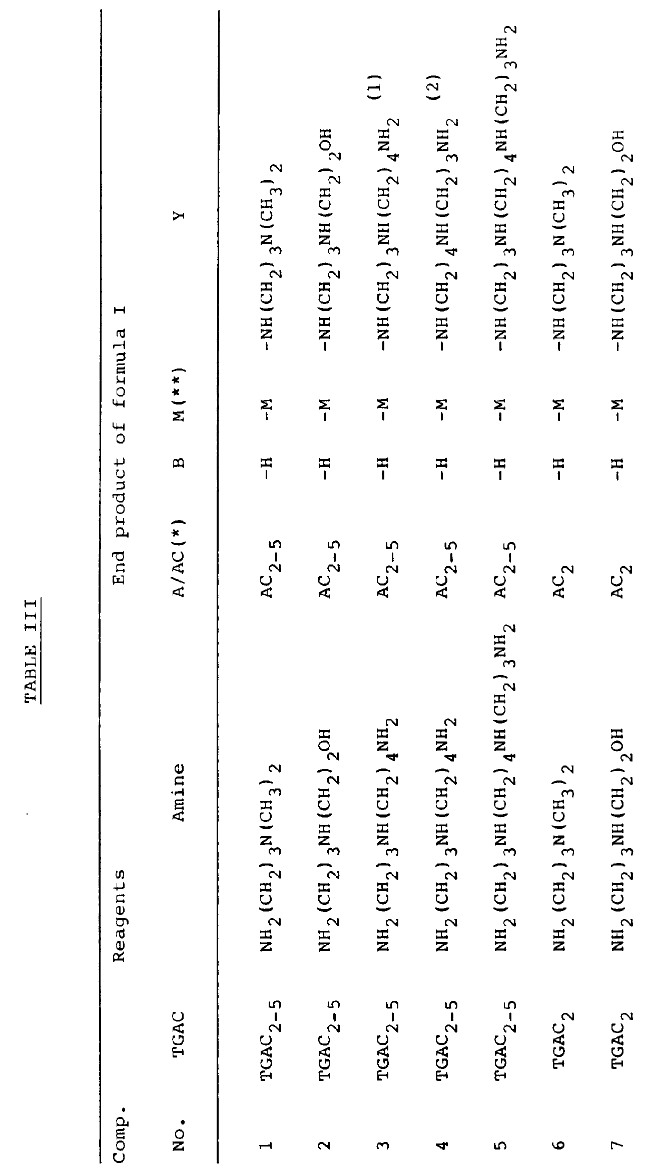

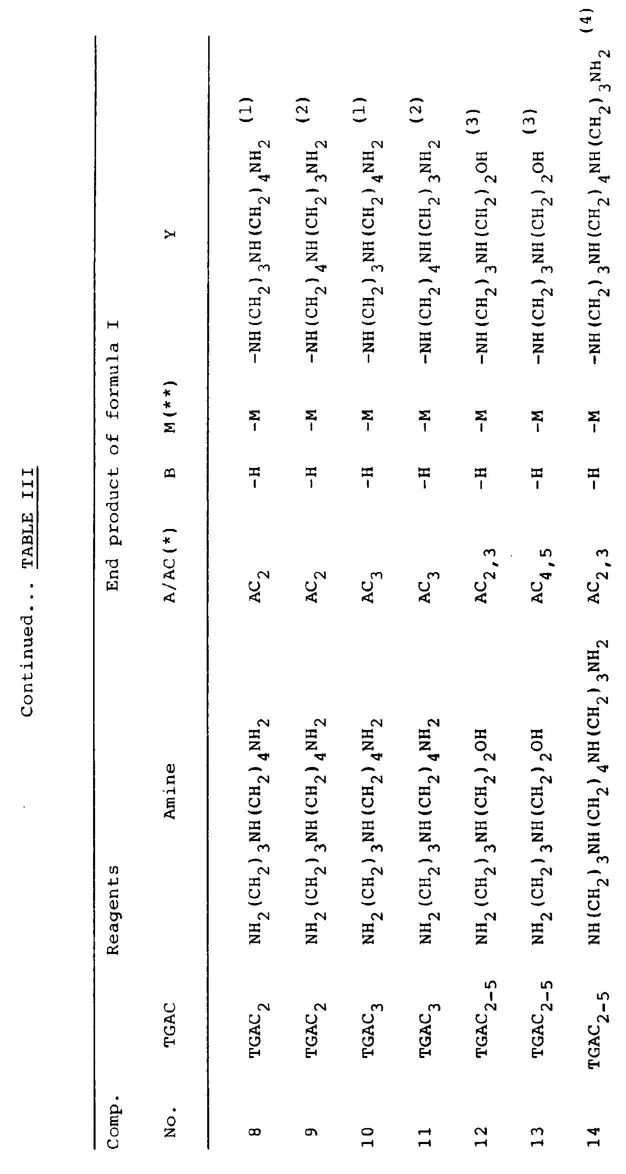

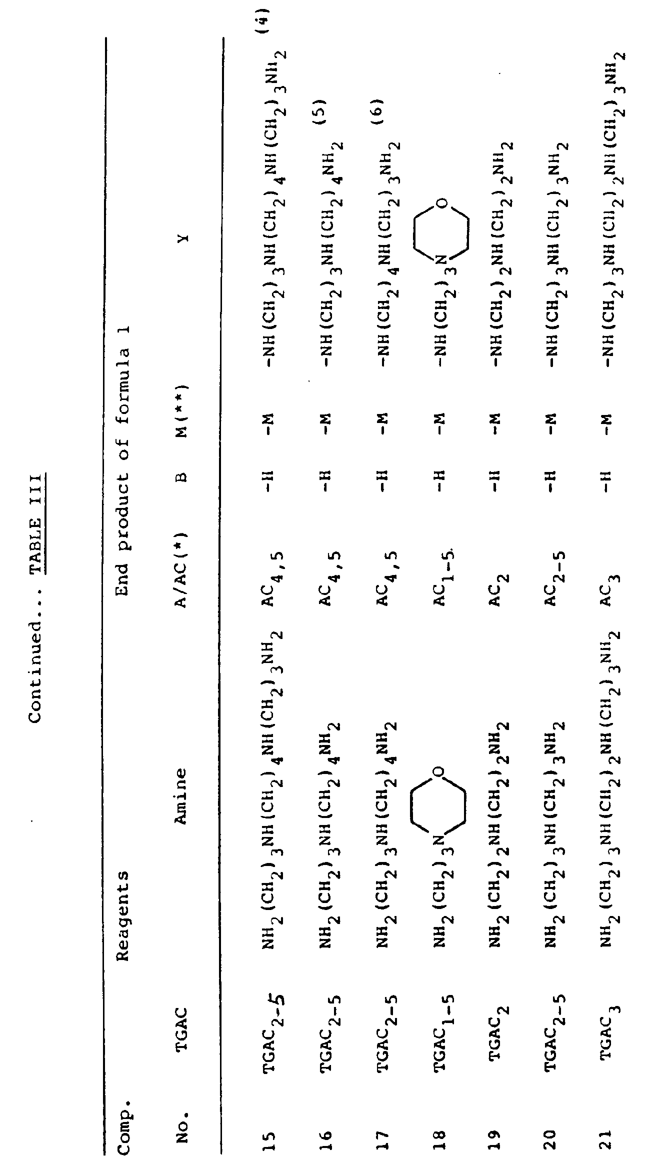

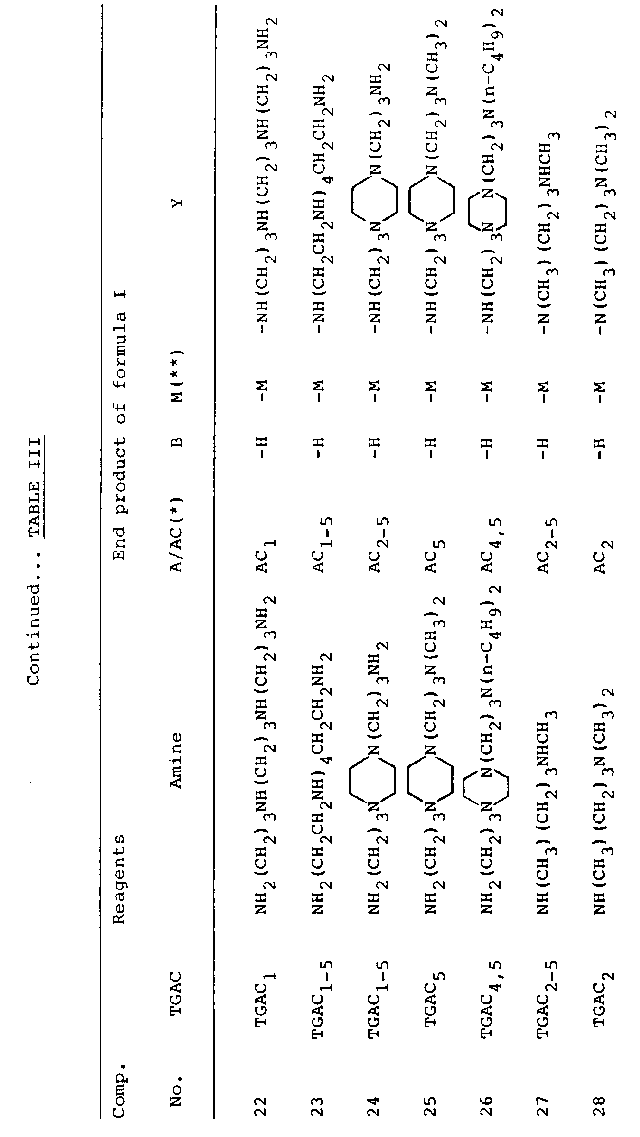

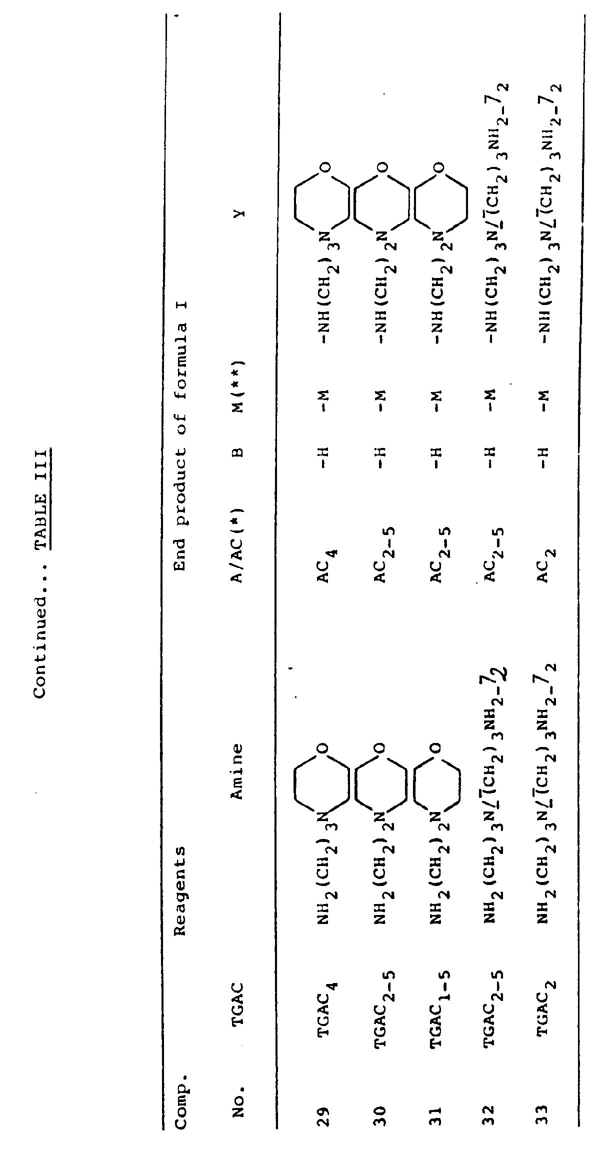

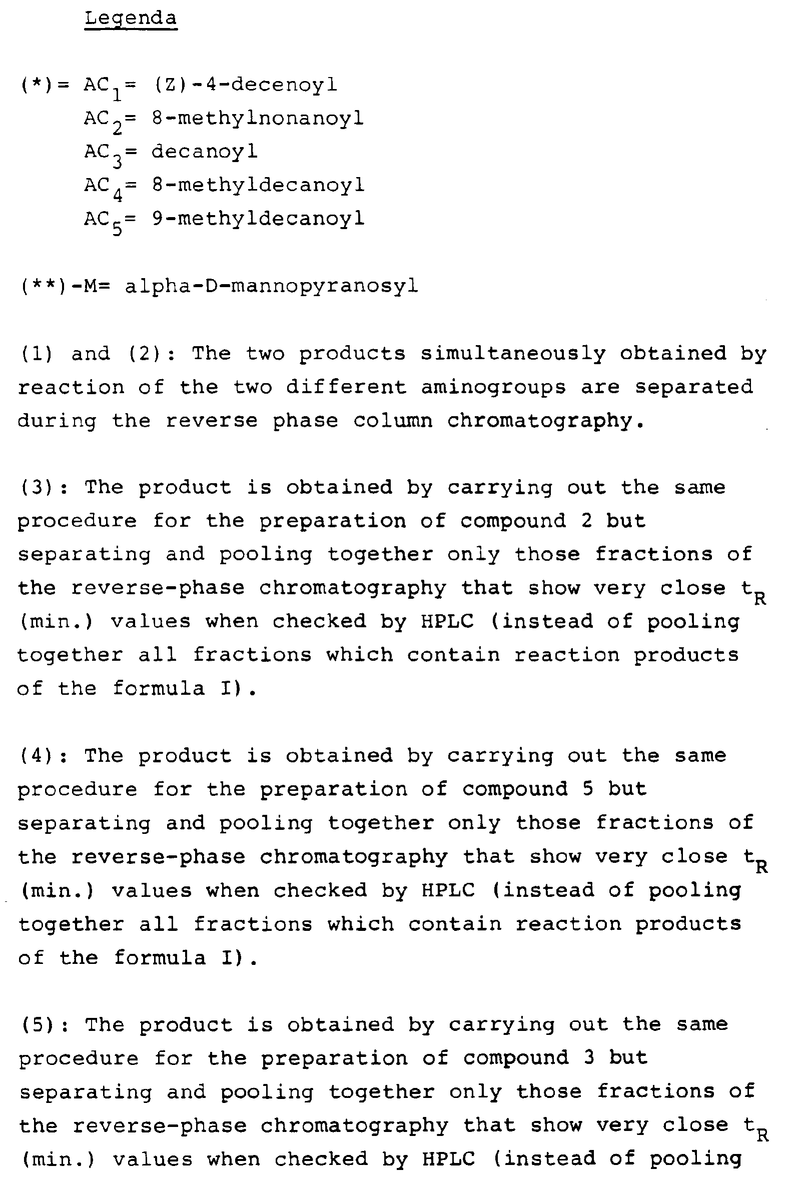

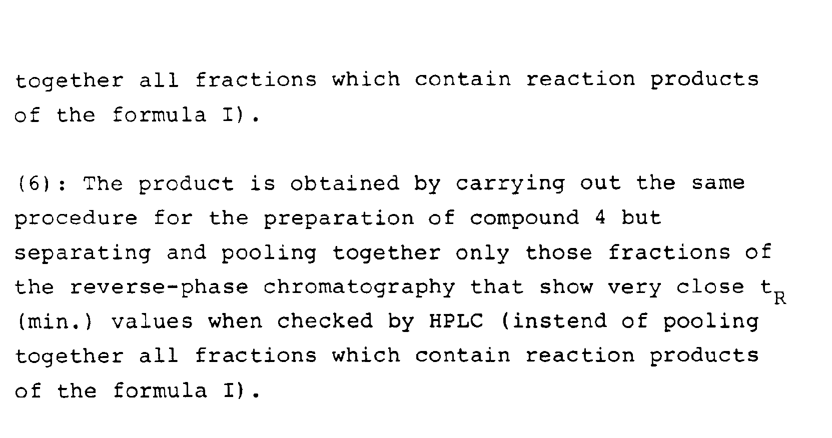

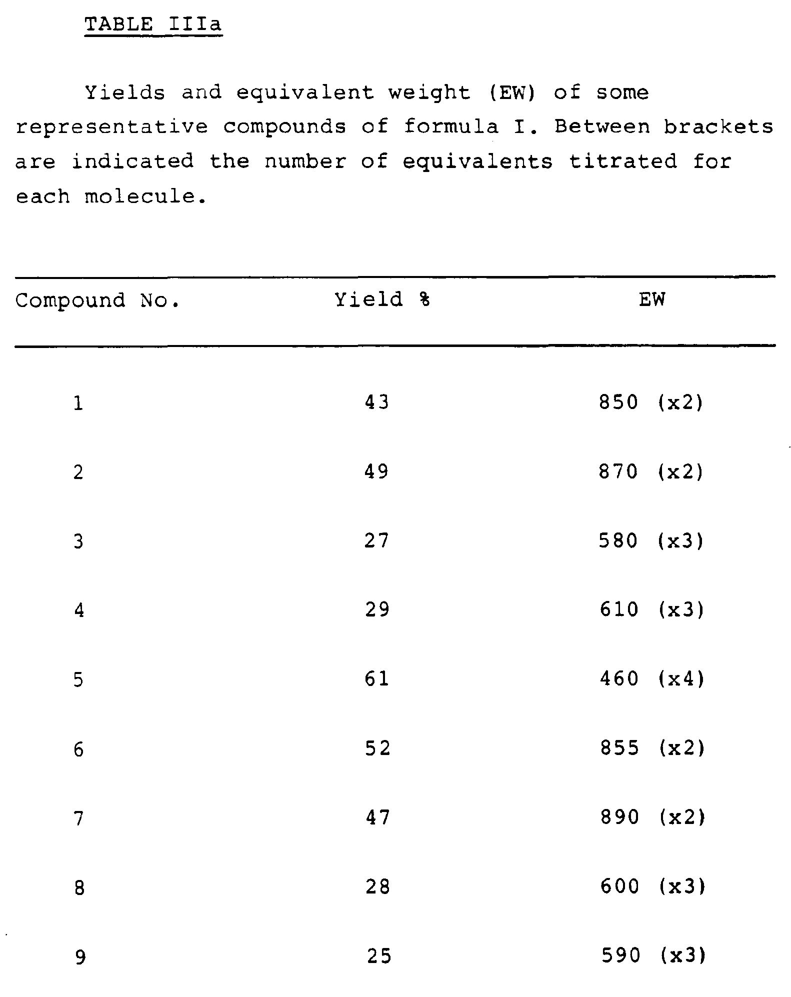

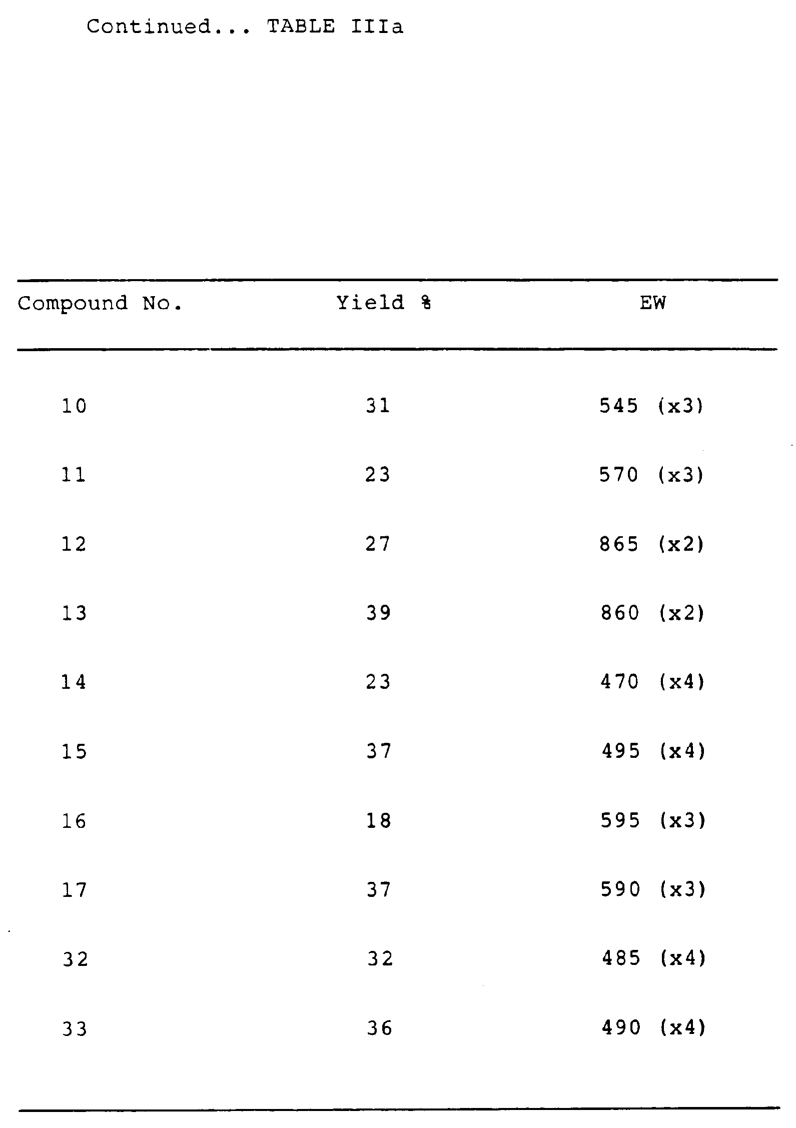

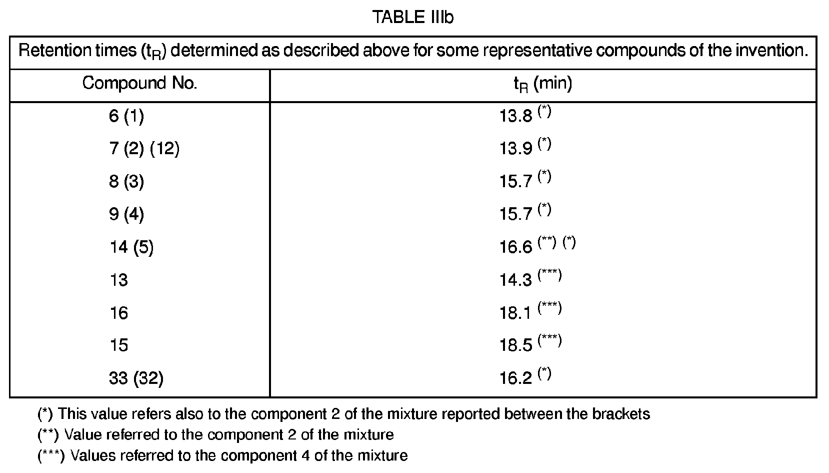

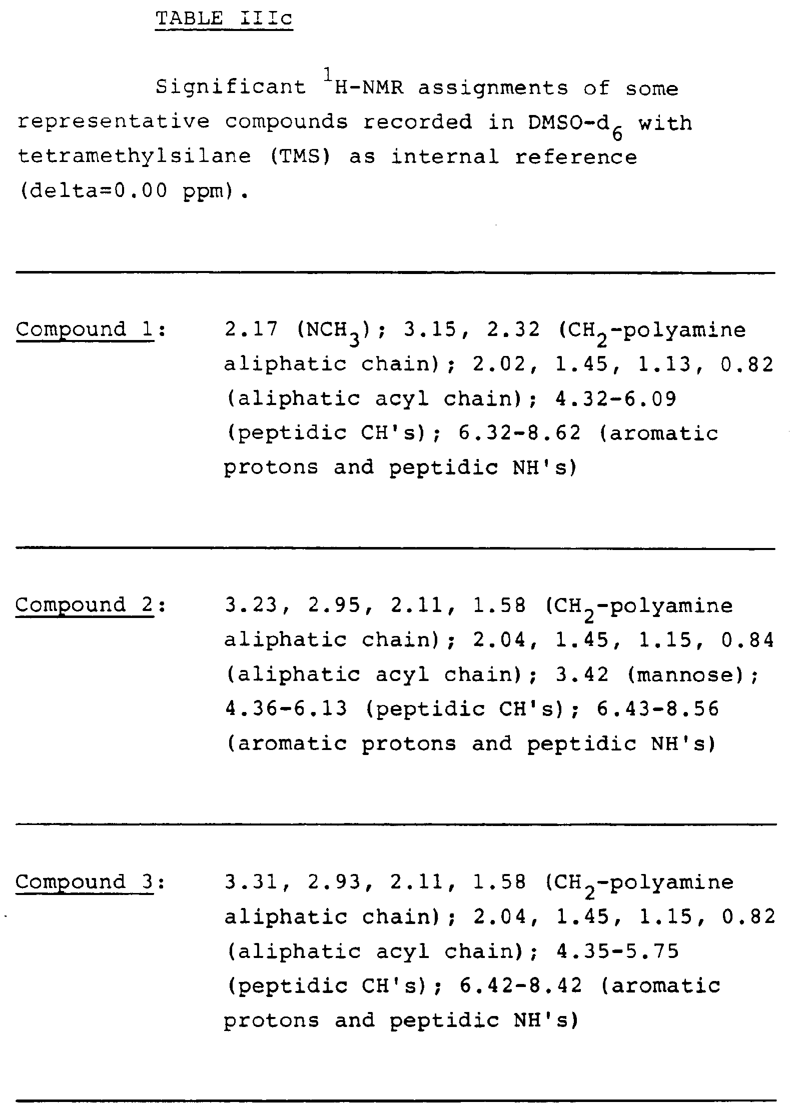

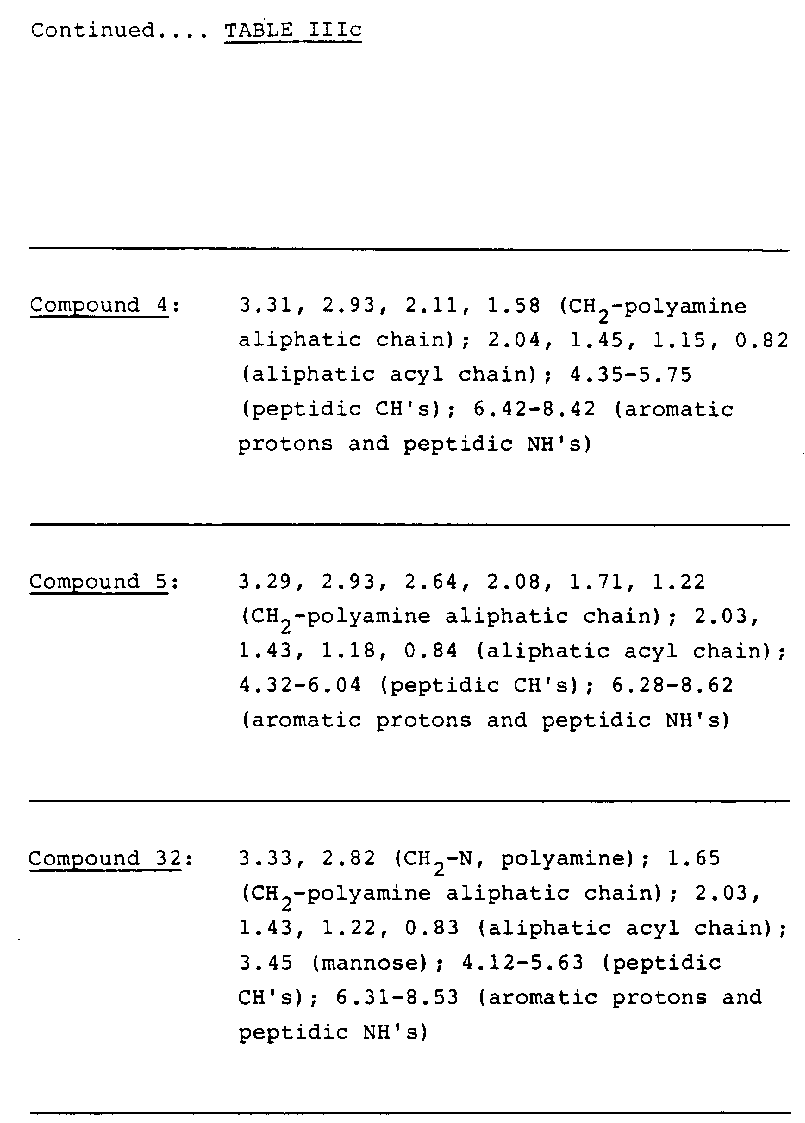

## Claims (Claims for the following Contracting State(s) : AT, BE, CH, DE, FR, GB, IT, LI, LU, NL, SE)

1. A teicoplanin derivative of the formula I 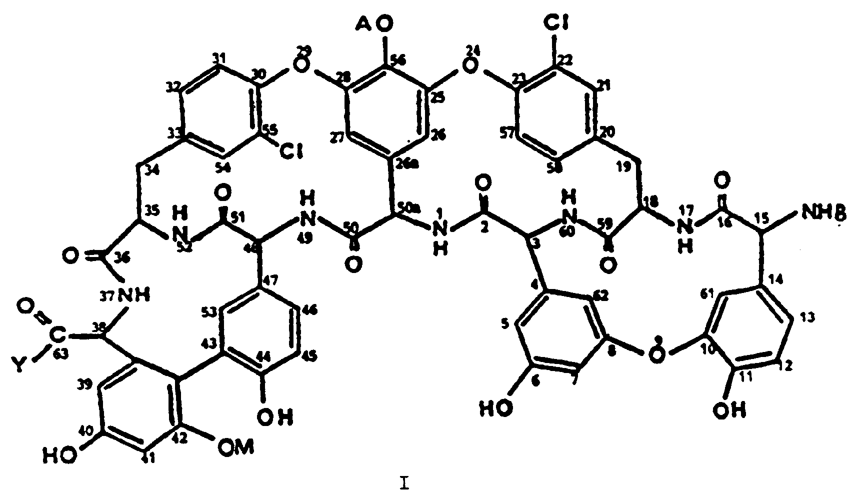 wherein:
A represents N[(C₉-C₁₂)aliphatic acyl]-beta-D-2-deoxy-2-aminoglucopyranosyl;
B is hydrogen or a protecting group of the amine function;
M represents alpha-D-mannopyranosyl;
Y represents a di- or poly-amine group of the formula 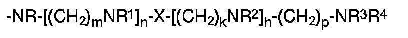 wherein:
R is hydrogen or linear or branched (C₁-C₈)alkyl;
R¹ is hydrogen or linear or branched (C₁-C₈)alkyl;
R² is hydrogen or linear or branched (C₁-C₈)alkyl;
R3 and R⁴ are each independently hydrogen, linear or branched (C₁-C₈)alkyl optionally bearing a NH₂, OH or SH substituent or taken together with the adjacent nitrogen atom, form a 5 to 7 membered saturated heterocyclic ring which may contain a further heteroatom selected from -S-, -O- and -NR⁵⁻ wherein R⁵ is hydrogen, (C₁-C₄)alkyl, phenyl, or phenyl-(C₁-C₄)alkyl
m, k and p each independently represent an integer from 2 to 8;
n and h, each independently, represent an integer from 0 to 4;
X represents a single bond, or when n is 1, taken together with the adjacent group NR¹, it may represent a bifunctional radical of the formula: 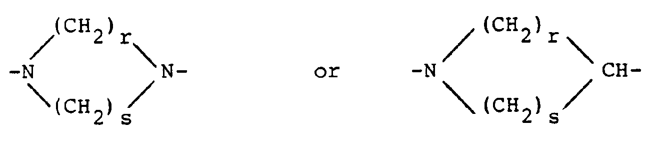
wherein r and s each independently represent an integer from 1 to 6 with the proviso that their sum is an integer from 3 to 8;
and its addition salts with acids.

2. A compound of claim 1 wherein the (C₉-C₁₂) aliphatic acyl radical of the moiety represented by the symbol A is: (Z)-4-decenoyl, 8-methylnonanoyl, decanoyl, 8-methyldecanoyl, 9-methyldecanoyl, 6-methyloctanoyl, nonanoyl, 10-methylundecanoyl or dodecanoyl.

3. A compound of claim 1 wherein A is as in claim 2, B is hydrogen or a protecting group of the amine function, R, R¹ and R² are hydrogen or linear or branched alkyl radicals of 1 to 4 carbon atoms, R³ and R⁴ are each independently hydrogen, linear or branched alkyl radicals of 1 to 4 carbon atoms optionally bearing a NH₂, OH or SH substituent, or R3 and R⁴ taken together with the adjacent nitrogen atom represent one of the following rings: pyrrolidine, piperidine, oxazolidine, thiazolidine, isoxazolidine, isothiazolidine, morpholine, piperazine, thiomorpholine, hexahydroazepine, hexahydro-1,5-diazepine and hexahydro-1,4-diazepine; R⁵ is hydrogen or Cₗ-C₄ alkyl;
the symbols m, k and p represent integers from 2 to 6, preferably, from 2 to 4;
the symbols n and h represent 0, 1 or 2, preferably 0 or 1;
the symbol X represents a single bond or, when n is 1, taken together with the adjacent group NR¹ represents a bifunctional radical of the formula 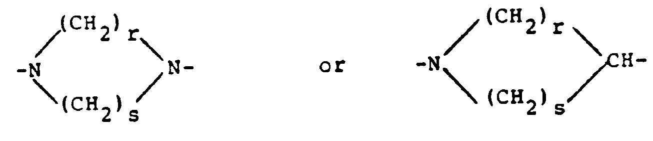
wherein r and s are both 2 or one is 1 and the other is 2 or 3;
and its addition salts with pharmaceutically acceptable acids.

4. A compound of claim 3 wherein the aliphatic acyl radical of the moiety represented by the symbol A is a (Cₗₒ-C₁₁)aliphatic acyl radical selected from: (Z)-4-decenoyl, 8-methylnonanoyl, decanoyl, 8-methyldecanoyl and 9-methyldecanoyl.

5. A compound of claim 4 wherein R is hydrogen or methyl, R¹ and R² are hydrogen, R³ and R⁴ each independently are hydrogen, a linear or branched alkyl of 1 to 4 carbon atoms optionally bearing a NH₂, OH or SH substituent or taken together with the adjacent nitrogen atom represents pyrrolidine, morpholine, or piperazine and R⁵ is hydrogen or methyl;
the symbols m, k and p represent integers from 2 to 4;
the symbols n and h represent 0 or 1;
the symbol X represents a single bond or when n is 1, taken together with the adjacent group NR¹ represents a bifunctional radical of the formula: 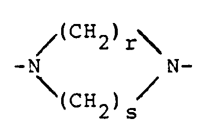 wherein
r and s are both 2;
and its addition salts with pharmaceutically acceptable acids.

6. A compound of claim 1 wherein
A represents N[(C₁₀-C₁₁)aliphatic acyl]-beta-D-2-deoxy-2-aminoglucopyranosyl wherein the (C₁₀-C₁₁)aliphatic acyl radical is selected from (Z)-4-decenoyl, 8-methylnonanoyl, decanoyl, 8-methyldecanoyl and 9-methyldecanoyl;
B is hydrogen or a protecting group of the amine function;
M represents alpha-D-mannopyranosyl;
Y represents a di- or poly-amine group of the formula:
-NH(CH₂)₃N(CH₃)₂;
-NH(CH₂)₃NH(CH₂)₂OH;
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂; 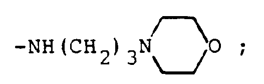
-NH(CH₂)₂NH(CH₂)₂NH₂;
-NH(CH₂)₃NH(CH₂)₃NH₂;
-NH(CH₂)₃NH(CH₂)₂NH(CH₂)₃NH₂;
-NH(CH₂)₃NH(CH₂)₃NH(CH₂)₃NH₂:
-NH(CH₂CH₂NH)₄CH₂CH_{z}NH₂; 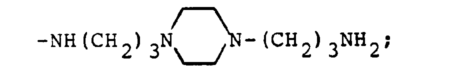 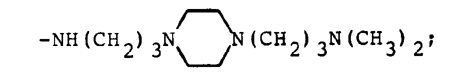 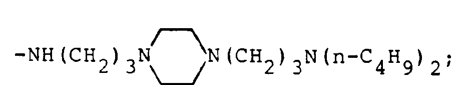
-N(CH₃)(CH₂)₃NHCH₃;
-N(CH₃)(CH₂)₃N(CH₃)₂; 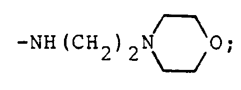
-NH(CH₂)₃N[(CH₂)₃NH₂]₂
and its addition salts with pharmaceutically acceptable acids.

7. A compound of claim 6 wherein Y represents
-NH(CH₂)₃NH(CH₂)₄NH₂
-NH(CH₂)₃NH(CH₂)₄NH(CH₂)₃NH₂ or
-NH(CH₂)₃N[(CH₂)₃NH₂]₂
and its addition salts with pharmaceutically acceptable acids.

8. A compound of claim 6 wherein the aliphatic acyl radical is 8-methylnonanoyl or decanoyl and Y represents
-NH(CH₂)₃NH(CH₂)₄NH₂
-NH(CH₂)₃NH(CH₂)₄NH(CH₂)₃NH₂ or
-NH(CH₂)₃N[(CH₂)₃NH₂]₂
and its addition salts with pharmaceutically acceptable acids.

9. A process for preparing a teicoplanin derivative of claim 1 which comprises amidation of the corresponding carboxylic teicoplanin starting material of the formula I wherein A, B, M have the same meanings as in claim 1 and Y is OH with an amine of the formula II 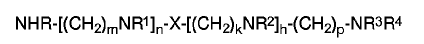 wherein R, R1, R², R³, R⁴, X, m, n, h, k and p have the same meanings as in claim 1 and, optionally, de-protecting the N15-amino function.

10. A process as in claim 9 where the amidation process is carried out by converting the carboxylic starting material in its corresponding activated ester preferably protected on the N¹⁵⁻amino function and the activated ester is reacted with a molar excess of an amine of the formula II in the presence of an organic polar solvent at a temperature between 5°C and 60°C preferably between 10°C and 30°C.

11. A substance as in any of claims 1 to 8 for use as a medicament.

12. Use of a substance as in any of claims 1 to 8 for the manufacture of a medicament for combatting infections caused by Group A Streptococci and/or coagulase negative Staphylococci.

13. A pharmaceutical formulation containing a compound of any of claims 1 to 8 as the active ingredient.

## Claims (Claims for the following Contracting State(s) : ES, GR)

1. A process for preparing a teicoplanin derivative of formula I 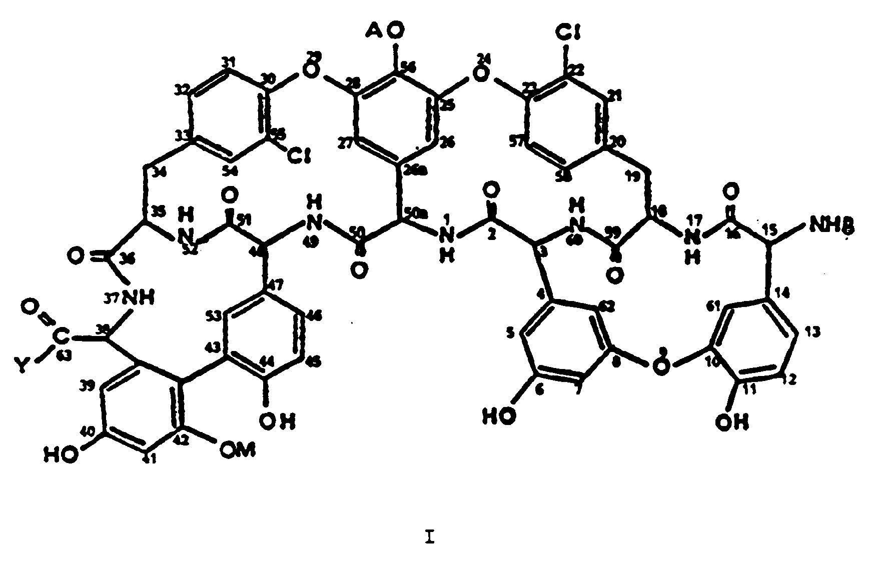 wherein:
A represents N[(C₉-C₁₂)aliphatic acyl]-beta-D-2-deoxy-2-aminoglucopyranosyl;
B is hydrogen or a protecting group of the amine function;
M represents alpha-D-mannopyranosyl;
Y represents a di- or poly-amine group of the formula 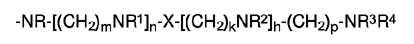 wherein:
R is hydrogen or linear or branched (C₁-C₈)alkyl;
R¹ is hydrogen or linear or branched (C₁-C₈)alkyl;
R² is hydrogen or linear or branched (C₁-C₈)alkyl;
R3 and R⁴ are each independently hydrogen, linear or branched (C₁-C₈)alkyl optionally bearing a NH₂, OH or SH substituent or taken together with the adjacent nitrogen atom, form a 5 to 7 membered saturated heterocyclic ring which may contain a further heteroatom selected from -S-, -0- and -NR⁵⁻ wherein R⁵ is hydrogen, (C₁-C₄)alkyl, phenyl, or phenyl-(C₁-C₄)alkyl
m, k and p each independently represent an integer from 2 to 8;
n and h, each independently, represent an integer from 0 to 4;
X represents a single bond, or when n is 1, taken together with the adjacent group NR¹, it may represent a bifunctional radical of the formula: 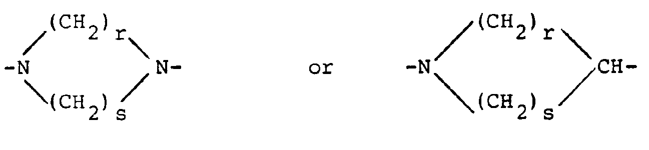 wherein r and s each independently represent an integer from 1 to 6 with the proviso that their sum is an integer from 3 to 8;
and its addition salts with acids,
which comprises amidation of the corresponding carboxylic teicoplanin starting material of the formula I wherein A, B, M have the same meanings as above and Y is OH with an amine of the formula II 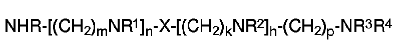 wherein R, R¹, R², R³, R⁴, X, m, n, h, k and p have the same meanings as above, and, optionally, de-protectin the N15-amino function.

2. A process as in claim 1 where the amidation process is carried out by converting the carboxylic starting material in its corresponding activated ester preferably protected on the N¹⁵⁻amino function and the activated ester is reacted with a molar excess of an amine of the formula II in the presence of an organic polar solvent at a temperature between 5°C and 60°C preferably between 10°C and 30°C.

3. A process as in claim 2 where the activated ester is the cyanomethyl ester and its molar proportion against the amine ranges from 1:10 to 1:20.

4. A process as in claim 3 where the cyanomethyl ester is prepared by reacting the carboxylic acid starting material preferably protected on the N¹⁵⁻amino function with an about 20 to 30-time molar excess of chloroacetonitrile in the presence of an inert organic solvent and a base which does not interfere with the reaction course at a temperature between 10°C and 60°C, preferably between 15 and 30°C.

5. A process as in claim 1 whereby the amidation process is carried out by reacting the carboxylic teicoplanin starting material with the amine of formula II in an inert organic solvent in the presence of a condensing agent selected from (C₁-C₄)alkyl, phenyl or heterocyclyl phosphorazidate at a temperature between 0°C and 20°C.

6. A process as in any of claims 1 to 4 for the manufacture of compounds of formula I wherein the (C₉-C₁₂-aliphatic acyl radicals of the moiety represented by the symbol A is:
(Z)-4-decenoyl, 8-methylnonanoyl, decanoyl, 8-methyldecanoyl, 9-methyldecanoyl, 6-methyloctanoyl, nonanoyl, 10-methylundecanoyl and dodecanoyl; B is hydrogen or a protecting group of the amine function, R, R¹ and R² are hydrogen or linear or branched alkyl radicals of 1 to 4 carbon atoms, R³ and R⁴ are each independently hydrogen, linear or branched alkyl radicals of 1 to 4 carbon atoms optionally bearing a NH₂, OH or SH substituent, or R³ and R⁴ taken together with the adjacent nitrogen atom represent one of the following rings:
pyrrolidine, piperidine, oxazolidine, thiazolidine, isoxazolidine, isothiazolidine, morpholine, piperazine, thiomorpholine, hexahydroazepine, hexahydro-1,5-diazepine and hexahydro-1,4-diazepine; R⁵ is hydrogen or Cₗ-C₄ alkyl;
the symbols m, k and p represent integers from 2 to 6, preferably, from 2 to 4;
the symbols n and h represent 0, 1 or 2, preferably 0 or 1;
the symbol X represents a single bond or, when n is 1, taken together with the adjacent group NR¹ represents a bifunctional radical of the formula 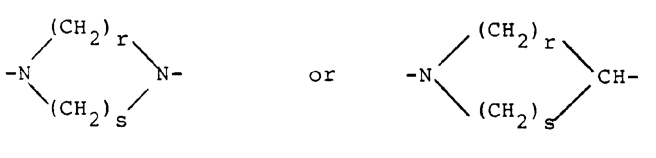
wherein r and s are both 2 or one is 1 and the other is 2 or 3;
and its addition salts with pharmaceutically acceptable acids.

7. A process as in any of claims 1 to 4 for the preparation of a compound of formula wherein the aliphatic acyl radical of the moiety represented by the symbol A is a (C₁₀-C₁₁)aliphatic acyl radical selected from: (Z)-4-decenoyl, 8-methylnonanoyl, decanoyl, 8-methyldecanoyl, 9-methyldecanoyl; B is hydrogen or a protecting group of the amine function;
R is hydrogen or methyl, R¹ and R² are hydrogen, R3 and R⁴ each independently are hydrogen, a linear or branched alkyl of 1 to 4 carbon atoms optionally bearing a NH₂, OH or SH substituent or taken together with the adjacent nitrogen atom represents pyrrolidine, morpholine, or piperazine and R⁵ is hydrogen or methyl;
the symbols m, k and _{Q} represent integers from 2 to 4;
the symbols n and h represent 0 or 1;
the symbol X represents a single bond or when n is 1, taken together with the adjacent group NR¹ represents a bifunctional radical of the formula: 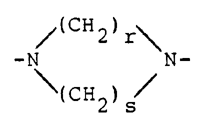
wherein r and s are both 2;
and its addition salts with pharmaceutically acceptable acids.

8. A process as in any of claims 1 to 4 for the manufacture of a compound of formula I wherein
A represents N[(C₁₀-C₁₁)aliphatic acyl]-beta-D-2-deoxy-2-aminoglucopyranosyl wherein the (Cᵢₒ-C₁₁)aliphatic acyl radical is selected from (Z)-4-decenoyl, 8-methylnonanoyl, decanoyl, 8-methyldecanoyl and 9-methyldecanoyl;
B is hydrogen or a protecting group of the amine function;
M represents alpha-D-mannopyranosyl;
Y represents a di- or poly-amine group of the formula:
-NH(CH₂)₃N(CH₃)₂;
-NH(CH₂)₃NH(CH₂)₂OH;
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂; 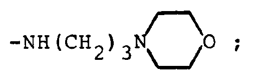
-NH(CH₂)₂NH(CH₂)₂NH₂;
-NH(CH₂)₃NH(CH₂)₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂CH₂NH)₄CH₂CH₂NH₂; 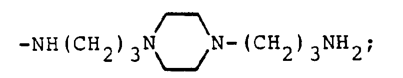 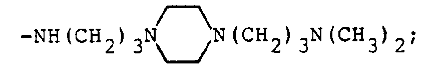 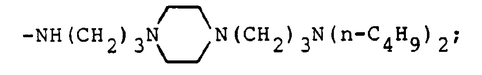
-N(CH₃)(CH₂)₃NHCH₃;
-N(CH₃)(CH₂)₃N(CH₃)₂; 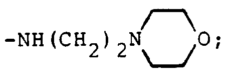
-NH(CH₂)₃N[(CH₂)₃NH₂]₂
and its addition salts with pharmaceutically acceptable acids.

9. A process as in any of claims 1 to 4 for the manufacture of a compound of formula I wherein A, B and M are as in
claim 8 and Y represents:
-NH(CH2) 3NH(CH2)4NH2
-NH(CH₂)₃NH(CH₂)₄NH(CH₂)₃NH₂ or
-NH(CH₂)₃N[(CH₂)₃NH₂]₂
and its addition salts with pharmaceutically acceptable acids.

10. A process as in any of claims 1 to 4 for the manufacture of a compound of formula I wherein the aliphatic acyl radical
of the moiety represented by the symbol A is 8-methylnonanoyl or decanoyl, B is hydrogen or a protecting group of
the amine function; M represents alpha-D-mannopyranosyl and Y represents:
-NH(CH₂)₃NH(CH₂)₄NH₂
-NH(CH₂)₃NH(CH₂)₄NH(CH₂)₃NH₂ or
-NH(CH₂)₃N[(CH₂)₃NH₂]₂
and its addition salts with pharmaceutically acceptable acids.

## Patentansprüche (Patentansprüche für folgende(n) Vertragsstaat(en) : AT, BE, CH, DE, FR, GB, IT, LI, LU, NL, SE)

1. Teicoplaninderivat der Formel 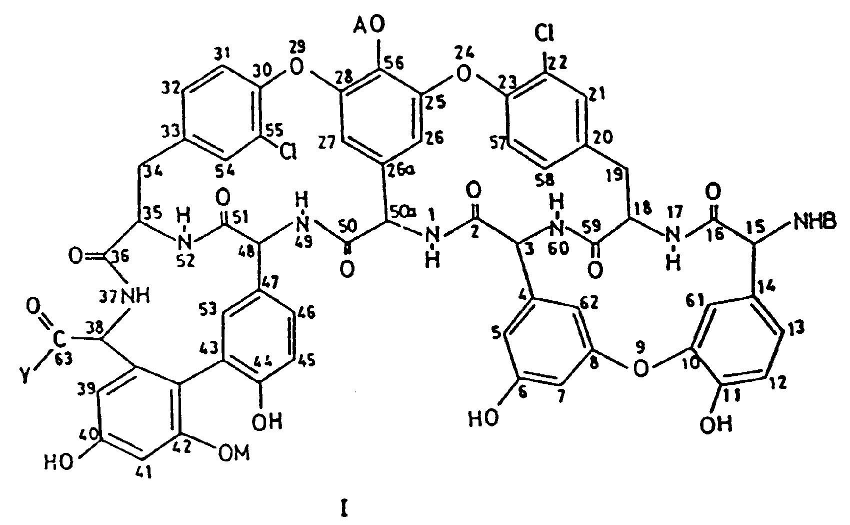 in der:
A einen N-[(C₉-C₁₂)aliphatischen-Acyl]-beta-D-2-deoxy-2-aminoglucapyranosylrest darstellt,
B ein Wasserstoffatom oder eine Schutzgruppe der Aminfunktion ist,
M eine alpha-D-Mannopyranosylgruppe darstellt,
Y einen Di- oder Polyaminrest der Formel 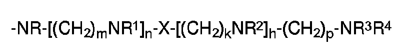
darstellt,
in der:
R ein Wasserstoffatom oder ein linearer oder verzweigter (C₁-C₈)-Alkylrest ist,
R¹ ein Wasserstoffatom oder ein linearer oder verzweigter (C₁-C₈)-Alkylrest ist,
R² ein Wasserstoffatom oder ein linearer oder verzweigter (C₁-Cₐ)-Alkylrest ist,
R3 und R⁴ jeweils unabhängig Wasserstoffatome, lineare oder verzweigte (C₁-C₈)-Akylreste darstellen, die gegebenenfalls einen NH₂-, OH- oder SH-Substituenten tragen oder zusammen mit dem benachbarten Stickstoffatom einen 5- bis 7-gliedrigen gesättigten heterocyclischen Ring bilden, der ein weiteres Heteroatom, ausgewählt aus - S-, -0- und -NR⁵⁻ enthalten kann, wobei R⁵ ein Wasserstoffatom, ein (C₁-C₄)-Alkylrest, eine Phenylgruppe oder ein Phenyl-(C₁-C₄)-alkylrest ist,
m, k und p jeweils unabhängig eine ganze Zahl von 2 bis 8 darstellen,
n und h jeweils unabhängig eine ganze Zahl von 0 bis 4 darstellen,
X eine Einfachbindung darstellt, oder, wenn n 1 ist, zusammen mit dem benachbarten Rest NR¹ einen bifunktionellen Rest der Formel: 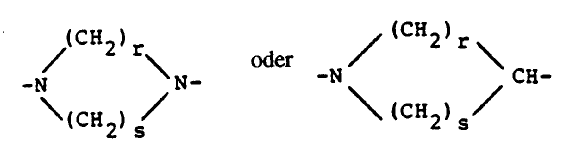 darstellen kann, in der r und s jeweils unabhängig eine ganze Zahl von 1 bis 6 darstellen, mit der Maßgabe, daß ihre Summe eine ganze Zahl von 3 bis 8 ist, und seine Additionssalze mit Säuren.

2. Verbindung nach Anspruch 1, in der der aliphatische (C₉-C₁₂)-Acylrest der durch das Symbol A dargestellten Einheit: eine (Z)-4-Decenoyl-, 8-Methylnonanoyl-, Decanoyl-, 8-Methyldecanoyl-, 9-Methyldecanoyl-, 6-Methyloctanoyl-, Nonanoyl-, 10-Methylundecanoyl- oder Dodecanoylgruppe ist.

3. Verbindung nach Anspruch 1, in der A die in Anspruch 2 angegebene Bedeutung hat, B ein Wasserstoffatom oder eine Schutzgruppe der Aminfunktion ist, R, R¹ und R² Wasserstoffatome oder lineare oder verzweigte Alkylreste mit 1 bis 4 Kohlenstoffatomen sind, R3 und R⁴ jeweils unabhängig Wasserstoffatome, lineare oder verzweigte Alkylreste mit 1 bis 4 Kohlenstoffatomen sind, die gegebenenfalls einen NH₂-, OH- oder SH-Substituenten tragen, oder R3 und R⁴ zusammen mit dem benachbarten Stickstoffatom einen der folgenden Ringe darstellen:
Pyrrolidin, Piperidin, Oxazolidin, Thiazolidin, Isoxazolidin, Isothiazolidin, Morpholin, Piperazin, Thiomorpholin, Hexahydroazepin, Hexahydro-1,5-diazepin und Hexahydro-1,4-diazepin; R⁵ ein Wasserstoffatom oder ein C₁-C₄-Alkylrest ist;
die Symbole m, k und p ganze Zahlen von 2 bis 6, vorzugsweise von 2 bis 4, darstellen;
die Symbole n und h 0, 1 oder 2, vorzugsweise 0 oder 1, darstellen;
das Symbol X eine Einfachbindung darstellt, oder, wenn n 1 ist, zusammen mit dem benachbarten Rest NR¹ einen bifunktionellen Rest der Formel 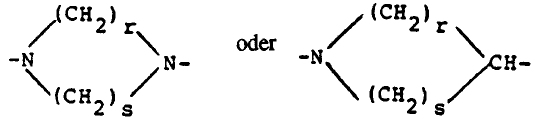
darstellt, in dem r und s beide die Zahl 2 darstellen oder eines die Zahl 1 darstellt und das andere 2 oder 3 ist;
und ihre Additionssalze mit pharmazeutisch verträglichen Säuren.

4. Verbindung nach Anspruch 3, in der der aliphatische Acylrest der durch das Symbol A dargestellten Einheit ein aliphatischer (C₁₀-C₁₁)-Acylrest, ausgewählt aus: (Z)-4-Decenoyl-, 8-Methylnonanoyl-, Decanoyl-, 8-Methyldecanoyl- und 9-Methyldecanoylgruppen, ist.

5. Verbindung nach Anspruch 4, in der R ein Wasserstoffatom oder eine Methylgruppe ist, R¹ und R² Wasserstoffatome sind, R3 und R⁴ jeweils unabhängig ein Wasserstoffatom, einen linearen oder verzweigten Alkylrest mit 1 bis 4 Kohlenstoffatomen darstellen, der gegebenenfalls einen NH₂-, OH- oder SH-Substituenten trägt, oder zusammen mit dem benachbarten Stickstoffatom eine Pyrrolidin-, Morpholin- oder Piperazingruppe darstellt, und R⁵ ein Wasserstoffatom oder eine Methylgruppe ist;
die Symbole m, k und p ganze Zahlen von 2 bis 4 darstellen;
die Symbole n und h 0 oder 1 darstellen;
das Symbol X eine Einfachbindung darstellt, oder, wenn n 1 ist, zusammen mit dem benachbarten Rest NR¹ einen bifunktionellen Rest der Formel: 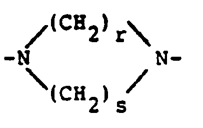
darstellt, in der r und s beide 2 sind;
und ihre Additionsalze mit pharmazeutisch verträglichen Säuren.

6. Verbindung nach Anspruch 1, in der
A einen N-[(C₁₀-C₁₁)aliphatischen-Acyl]-beta-D-2-deoxy-2-aminoglucopyranosylrest darstellt, in dem der aliphatische (C₁₀-C₁₁)-Acylrest ausgewählt ist aus (Z)-4-decenoyl-, 8-Methylnonanoyl-, Decanoyl-, 8-Methyldecanoyl- und 9-Methyldecanoylgruppen;
B ein Wasserstoffatom oder eine Schutzgruppe der Aminfunktion ist;
M eine alpha-D-Mannopyranosylgruppe darstellt;
Y einen Di- oder Polyaminrest der Formel:
-NH(CH₂)₃N(CH₃)₂;
-NH(CH₂)₃NH(CH₂)₂OH:
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂; 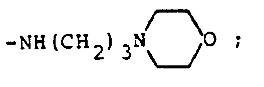
-NH(CH₂)₂NH(CH₂)₂NHz:
-NH(CH₂)₃NH(CH₂)₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂CH₂NH)₄CH₂CH₂NH_{z}; 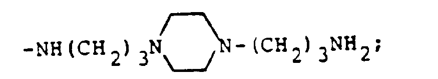 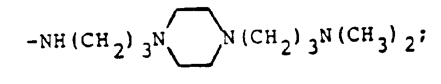 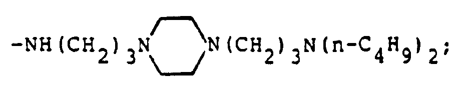
-N(CH₃)(CH₂)₃NHCH₃;
-N(CH₃)(CH₂)₃N(CH₃)₂; 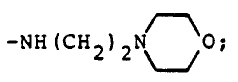
-NH(CH₂)₃N[(0H₂)₃NH₂]₂
darstellt,
und ihre Additionssalze mit pharmazeutisch verträglichen Säuren.

7. Verbindung nach Anspruch 6, in der Y
-NH(CH₂)₃NH(CH₂)₄NH₂
-NH(CH₂)₃NH(CH₂)₄NH(CH₂)₃NH₂ oder
-NH(CH₂)₃N[(CH₂)₃NH₂]₂
darstellt, und ihre Additionssalze mit pharmazeutisch verträglichen Säuren.

8. Verbindung nach Anspruch 6, in der der aliphatische Acylrest eine 8-Methylnonanoyl-oder Decanoylgruppe ist und Y
-NH (CH₂)₃NH (CH₂)₄NH₂
-NH (CH₂)₃NH (CH₂)₄NH(CH₂)₃NH₂ oder
-NH(CH₂)₃N[(CH₂)₃NH₂]₂
darstellt, und ihre Additionssalze mit pharmazeutisch verträglichen Säuren.

9. Verfahren zur Herstellung eines Teicoplaninderivats nach Anspruch 1, umfassend die Amidierung der entsprechenden Teicoplaninausgangssubstanz der Formel 1, in der A, B, M die gleiche Bedeutung wie in Anspruch 1 haben und Y OH ist, mit einem Amin der Formel II 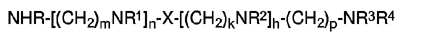 in der R, R1, R², R3, R⁴, X, m, n, h, k und p die in Anspruch 1 angegebenen Bedeutungen haben und gegebenenfalls Schutzgruppenabspaltung Von der N¹⁵⁻Aminofunktion.

10. Verfahren nach Anspruch 9, bei dem das Amidierungsverfahren unter Umwandeln der Carbonsäureausgangssubstanz in ihren entsprechenden aktivierten Ester, vorzugsweise an der N¹⁵⁻Aminofunktion geschützt, durchgeführt und der aktivierte Ester mit einem molaren Überschuß eines Amins der Formel II in Gegenwart eines organischen polaren Lösungsmittels bei einer Temperatur zwischen 5°C und 60°C, vorzugsweise zwischen 10°C und 30°C, umgesetzt wird.

11. Substanz nach einem der Ansprüche 1 bis 8 zur Verwendung als Medikament.

12. Verwendung einer Substanz nach einem der Ansprüche 1 bis 8 zur Herstellung eines Medikaments zur Bekämpfung von Infektionen, die durch Streptococcen des Typs A und/oder Koagulase-negative Staphylococcen verursacht werden.

13. Arzneimittel, das eine Verbindung nach einem der Ansprüche 1 bis 8 als Wirkstoff enthält.

## Patentansprüche (Patentansprüche für folgende(n) Vertragsstaat(en) : ES, GR)

1. Verfahren zur Herstellung eines Teicoplaninderivats der Formel 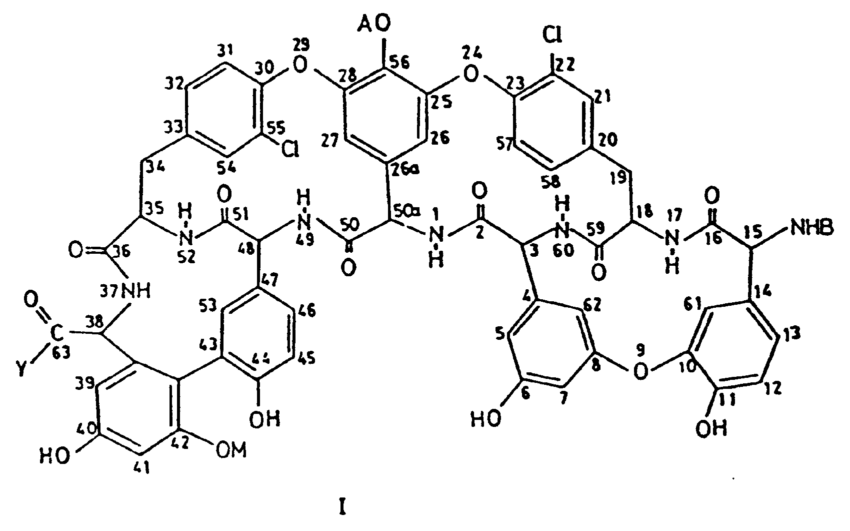 in der:
A einen N-[(C₉-C₁₂)aliphatischen-Acyl]-beta-D-2-deoxy-2-aminoglucopyranosylrest darstellt,
B ein Wasserstoffatom oder eine Schutzgruppe der Aminfunktion ist,
M eine alpha-D-Mannopyranosylgruppe darstellt,
Y einen Di- oder Polyaminrest der Formel 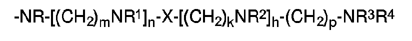
darstellt,
in der:
R ein Wasserstoffatom oder ein linearer oder verzweigter (C₁-Cₛ)-Alkylrest ist,
R¹ ein Wasserstoffatom oder ein linearer oder verzweigter (C₁-C₈)-Alkylrest ist,
R² ein Wasserstoffatom oder ein linearer oder verzweigter (C₁-C₈)-Alkylrest ist,
R3 und R⁴ jeweils unabhängig Wasserstoffatome, lineare oder verzweigte (C₁-C₈)-Alkylreste darstellen, die gegebenenfalls einen NH_{z}-, OH- oder SH-Substituenten tragen oder zusammen mit dem benachbarten Stickstoffatom einen 5- bis 7-gliedrigen gesättigten heterocyclischen Ring bilden, der ein weiteres Heteroatom, ausgewählt aus -S-, -O- und -NR⁵⁻ enthalten kann, wobei R⁵ ein Wasserstoffatom, ein (C₁-C₄)-Alkylrest, eine Phenylgruppe oder ein Phenyl-(C₁-C₄)-alkylrest ist,
m, k und p jeweils unabhängig eine ganze Zahl von 2 bis 8 darstellen,
n und h jeweils unabhängig eine ganze Zahl von 0 bis 4 darstellen,
X eine Einfachbindung darstellt, oder, wenn n 1 ist, zusammen mit dem benachbarten Rest NR¹ einen bifunktionellen Rest der Formel: 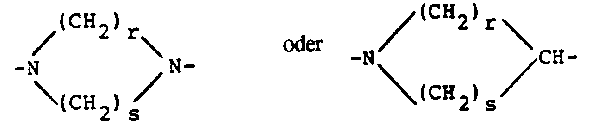 darstellen kann, in der r und s jeweils unabhängig eine ganze Zahl von 1 bis 6 darstellen, mit der Maßgabe, daß ihre Summe eine ganze Zahl von 3 bis 8 ist, und seiner Additionssalze mit Säuren,
umfassend die Amidierung der entsprechenden Teicoplaninausgangssubstanz der Formel in der A, B, M die gleiche Bedeutung wie vorstehend haben und Y OH ist, mit einem Amin der Formel II 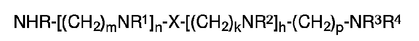 in der R, R1, R², R3, R⁴, X, m, n, h, k und p die vorstehend angegebenen Bedeutungen haben und gegebenenfalls Schutzgruppenabspaltung von der N¹⁵⁻Aminofunktion.

2. Verfahren nach Anspruch 1, wobei das Amidierungsverfahren unter Umwandeln der Carbonsäureausgangssubstanz in ihren entsprechenden aktivierten Ester, vorzugsweise an der N¹⁵⁻Aminofunktion geschützt, durchgeführt und der aktivierte Ester mit einem molaren Überschuß eines Amins der Formel II in Gegenwart eines organischen polaren Lösungsmittels bei einer Temperatur zwischen 5°C und 60°C, vorzugsweise zwischen 10°C und 30°C, umgesetzt wird.

3. Verfahren nach Anspruch 2, wobei der aktivierte Ester der Cyanomethylester ist und sein Molverhältnis zum Amin 1:10 bis 1:20 beträgt.

4. Verfahren nach Anspruch 3, wobei der Cyanomethylester durch Umsetzung der Carbonsäureausgangssubstanz, vorzugsweise an der N¹⁵⁻Aminofunktion geschützt, mit einem etwa 20 bis 30-fachen molaren Überschuß von Chloracetonitril in Gegenwart eines inerten organischen Lösungsmittels und einer Base, die nicht in den Reaktionsverlauf eingreift, bei einer Temperatur zwischen 10°C und 60°C, vorzugsweise zwischen 15 und 30°C, hergestellt wird.

5. Verfahren nach Anspruch 1, wobei das Amidierungsverfahren durch Umsetzung der Carbonsäure-Teicoplaninausgangssubstanz mit dem Amin der Formel 11 in einem inerten organischen Lösungsmittel in Gegenwart eines Kondensationsmittels, ausgewählt aus einem (C₁-C₄)-Alkyl-, Phenyl- oder heterocyclischen Phosphorazidat bei einer Temperatur zwischen 0°C und 20°C durchgeführt wird.

6. Verfahren nach einem der Ansprüche 1 bis 4 zur Herstellung von Verbindungen der Formel in der der aliphatische (C₉-C₁₂-Acylrest der durch das Symbol A dargestellten Einheit:
eine (Z)-4-Decenoyl-, 8-Methylnonanoyl-, Decanoyl-, 8-Methyldecanoyl-, 9-Methyldecanoyl-, 6-Methyloctanoyl-, Nonanoyl-, 10-Methylundecanoyl- oder Dodecanoylgruppe ist; B ein Wasserstoffatom oder eine Schutzgruppe der Aminfunktion ist, R, R¹ und R² Wasserstoffatome oder lineare oder verzweigte Alkylreste mit 1 bis 4 Kohlenstoffatomen sind, R³ und R⁴ jeweils unabhängig Wasserstoffatome, lineare oder verzweigte Alkylreste mit 1 bis 4 Kohlenstoffatomen sind, die gegebenenfalls einen NH_{z}-, OH- oder SH-Substituenten tragen, oder R³ und R⁴ zusammen mit dem benachbarten Stickstoffatom einen der folgenden Ringe darstellen:
Pyrrolidin, Piperidin, Oxazolidin, Thiazolidin, Isoxazolidin, Isothiazolidin, Morpholin, Piperazin, Thiomopholin, Hexahydroazepin, Hexahydro-1,5-diazepin und Hexahydro-1,4-diazepin; R⁵ ein Wasserstoffatom oder ein C₁-C₄-Alkylrest ist;
die Symbole m, k und p ganze Zahlen von 2 bis 6, vorzugsweise von 2 bis 4, darstellen;
die Symbole n und h 0, 1 oder 2, vorzugsweise 0 oder 1, darstellen;
das Symbol X eine Einfachbindung darstellt, oder, wenn n 1 ist, zusammen mit dem benachbarten Rest NR¹ einen bifunktionellen Rest der Formel 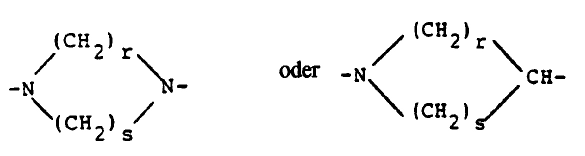
darstellt, in dem r und s beide die Zahl 2 darstellen oder eines die Zahl 1 darstellt und das andere 2 oder 3 ist; und ihrer Additionssalze mit pharmazeutisch verträglichen Säuren.

7. Verfahren nach einem der Ansprüche 1 bis 4 zur Herstellung einer Verbindung der Formel in der der aliphatische Acylrest der durch das Symbol A dargestellten Einheit ein aliphatischer (C₁₀-C_{1 1})-Acylrest, ausgewählt aus: (Z)-4-Decenoyl-, 8-Methylnonanoyl-, Decanoyl-, 8-Methyldecanoyl- und 9-Methyldecanoylgruppen ist; B ein Wasserstoffatom oder eine Schutzgruppe der Aminfunktion ist; R ein Wasserstoffatom oder eine Methylgruppe ist, R¹ und R² Wasserstoffatome sind, R3 und R⁴ jeweils unabhängig ein Wasserstoffatom, einen linearen oder verzweigten Alkylrest mit 1 bis 4 Kohlenstoffatomen darstellen, der gegebenenfalls einen NH₂-, OH- oder SH-Substituenten trägt oder zusammen mit dem benachbarten Stickstoffatom eine Pyrrolidin-, Morpholin- oder Piperazingruppe darstellt, und R⁵ ein Wasserstoffatom oder eine Methylgruppe ist;
die Symbole m, k und p ganze Zahlen von 2 bis 4 darstellen;
die Symbole n und h 0 oder 1 darstellen;
das Symbol X eine Einfachbindung darstellt, oder, wenn n 1 ist, zusammen mit dem benachbarten Rest NR¹ einen bifunktionellen Rest der Formel: 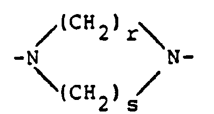
darstellt, in der r und s beide 2 sind;
und ihrer Additionssalze mit pharmazeutisch verträglichen Säuren.

8. Verfahren nach einem der Ansprüche 1 bis 4 zur Herstellung einer Verbindung der Formel in der
A einen N-[(C₁₀-C₁₁)aliphatischen-Acyl]-beta-D-2-deoxy-2-aminoglucopyranosylrest darstellt, in dem der aliphatische (C₁₀-C₁₁)-Acylrest ausgewählt ist aus (Z)-4-decenoyl-, 8-Methylnonanoyl-, Decanoyl-, 8-Methyldecanoyl- und 9-Methyldecanoylgruppen;
B ein Wasserstoffatom oder eine Schutzgruppe der Aminfunktion ist;
M eine alpha-D-Mannopyranosylgruppe darstellt;
Y einen Di- oder Polyaminrest der Formel:
-NH(CH₂)₃N(CH₃)₂;
-NH(CH₂)₃NH(CH₂)₂OH;
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂; 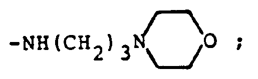
-NH(CH₂)₂NH(CH₂)₂NH₂;
-NH(CH₂)₃NH(CH₂)₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂CH₂NH)₄CH₂CH₂NH₂; 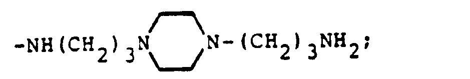 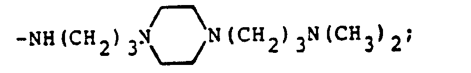 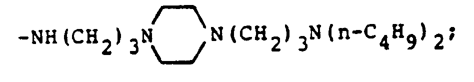
-N(CH₃)(CH₂)₃NHCH₃;
-N(CH₃)(CH₂)₃N(CH₃)₂; 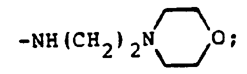
-NH(CH₂)₃N[(CH₂)₃NH₂]₂
darstellt,
und ihrer Additionssalze mit pharmazeutisch verträglichen Säuren.

9. Verfahren nach einem der Ansprüche 1 bis 4 zur Herstellung einer Verbindung der Formel I, in der A, B und M die in Anspruch 8 angegebene Bedeutung haben und Y
-NH(CH₂)₃NH(CH₂)₄NH₂
-NH(CH₂)₃NH(CH₂)₄NH(CH₂)₃NH₂ oder
-NH(CH₂)₃N[(CH₂)₃NH_{2]2}
darstellt, und ihrer Additionssalze mit pharmazeutisch verträglichen Säuren.

10. Verfahren nach einem der Ansprüche 1 bis 4 zur Herstellung einer Verbindung der Formel I, in der der aliphatische Acylrest der durch das Symbol A dargestellten Einheit eine 8-Methylnonanoyl- oder Decanoylgruppe ist, B ein Wasserstoffatom oder eine Schutzgruppe der Aminfunktion darstellt; M eine alpha-D-Mannopyranosylgruppe darstellt und Y
-NH(CH₂)₃NH (CH₂)₄NH₂
-NH(CH₂)₃NH(CH₂)₄NH(CH₂)₃NH₂ oder
-NH(CH₂)₃N[(CH₂)₃NH₂]₂
darstellt, und ihrer Additionsalze mit pharmazeutisch verträglichen Säuren.

## Revendications (Revendications pour l'(les) Etat(s) contractant(s) suivant(s) : AT, BE, CH, DE, FR, GB, IT, LI, LU, NL, SE)

1. Dérivé de téicoplanine de formule 1 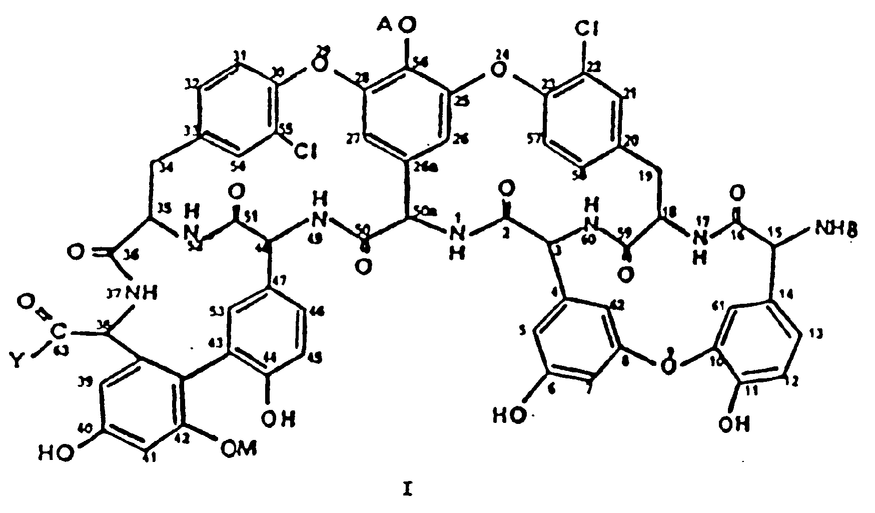 dans laquelle
A représente un groupememt N-[acyl(C₉-C₁₂) aliphatique]-β-D-2-désoxy-2-aminoglucopyrannosyle;
B est un atome d'hydrogène ou un groupe protecteur de la fonction amine;
M représente le groupe a-D-mannopyrannosyle,
Y représente un groupe di- ou polyamino de formule 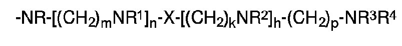 dans laquelle
R est un atome d'hydrogène ou un groupe alkyle en C₁-C₈ linéaire ou ramifié;
R¹ est un atome d'hydrogène ou un groupe alkyle en C₁-C₈ linéaire ou ramifié;
R² est un atome d'hydrogène ou un groupe alkyle en Ci-Ce linéaire ou ramifié;
R3 et R⁴ sont, indépendamment l'un de l'autre, un atome d'hydrogène ou un groupe alkyle en C₁-C₈ linéaire ou ramifié, portant éventuellement un substituant NH₂, OH ou SH, ou pris ensemble, avec l'atome d'azote contigu, forment un cycle hétérocyclique saturé à 5-7 chaînons pouvant contenir un autre hétéroatome choisi parmi -S-, -0- et -NR⁵-, R⁵ étant un atome d'hydrogène ou un groupe alkyle en C₁-C₄, phényle ou phénylalkyle en Ci-C₄;
m, k et p représentent, indépendamment les uns des autres, un nombre entier allant de 2 à 8;
n et h représentent, indépendamment l'un de l'autre, un nombre entier allant de 0 à 4;
X représente une simple liaison, ou, lorsque n est 1, peut représenter, conjointement avec le groupe contigu NR1, un radical bifonctionnel de formule 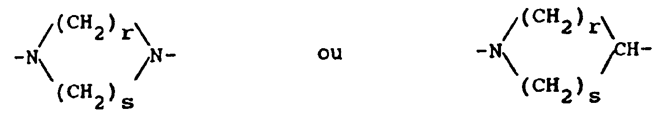 r et s représentant, indépendamment l'un de l'autre, un nombre entier allant de 1 à 6, étant entendu que leur somme est un nombre entier allant de 3 à 8;
et ses sels d'addition avec des acides.

2. Composé selon la revendication 1, dans lequel le radical acyle aliphatique en C₉-C₁₂ du fragment représenté par le symbole A est:
le groupe (Z)-4-décénoyle, 8-méthylnonanoyle, décanoyle, 8-méthyldécanoyle, 9-méthyldécanoyle, 6-méthylocta- noyle, nonanoyle, 10-méthylundécanoyle ou dodécanoyle.

3. Composé selon la revendication 1, dans lequel A est tel que dans la revendication 2, B est un atome d'hydrogène ou un groupe protecteur de la fonction amine, R, R¹ et R² sont des atomes d'hydrogène ou des radicaux alkyle linéaires ou ramifiés ayant de 1 à 4 atomes de carbone, R3 et R⁴ sont, indépendamment l'un de l'autre, un atome d'hydrogène ou un radical alkyle linéaire ou ramifié ayant de 1 à 4 atomes de carbone, portant éventuellement un substituant NH₂, OH ou SH, ou R³ et R⁴ pris ensemble, avec l'atome d'azote contigu, représentent l'un des cycles suivants: pyrrolidine, pipéridine, oxazolidine, thiazolidine, isoxazolidine, isothiazolidine, morpholine, pipérazine, thiomorpholine, hexahydroazépine, hexahydro-1,5-diazépine et hexahydro-1,4-diazépine; R⁵ est un atome d'hydrogène ou un groupe alkyle en Ci-C₄;
les symboles m, k et p représentent des nombres entiers allant de 2 à 6, de préférence de 2 à 4;
les symboles n et h représentent 0, 1 ou 2, de préférence 0 ou 1;
le symbole X représente une simple liaison, ou, lorsque n est 1, pris ensemble avec le groupe contigu NR¹ représente un radical bifonctionnel de formule 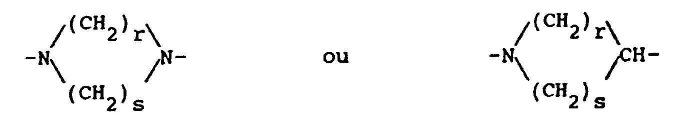
r et s étant l'un et l'autre 2 ou l'un étant 1 et l'autre étant 2 ou 3;
et ses sels d'addition avec des acides pharmaceutiquement acceptables.

4. Composé selon la revendication 3, dans lequel le radical acyle aliphatique du fragment représenté par le symbole A est un radical acyle aliphatique en C₁₀-C₁₁ choisi parmi les radicaux (Z)-4-décénoyle, 8-méthylnonanoyle, décanoyle, 8-méthyldécanoyle et 9-méthyldécanoyle.

5. Composé selon la revendication 4, dans lequel R est un atome d'hydrogène ou le groupe méthyle, R¹ et R² sont des atomes d'hydrogène, R³ et R⁴ sont, indépendamment l'un de l'autre, un atome d'hydrogène ou un groupe alkyle linéaire ou ramifié ayant de 1 à 4 atomes de carbone, portant éventuellement un substituant NH₂, OH ou SH, ou pris ensemble avec l'atome d'azote contigu, représentent un cycle pyrrolidine, morpholine ou pipérazine, et R⁵ est un atome d'hydrogène ou le groupe méthyle;
les symboles m, k et p représentent des nombres entiers allant de 2 à 4;
les symboles n et h représentent 0 ou 1 ;
le symbole X représente une simple liaison, ou, lorsque n est 1, pris ensemble avec le groupe contigu NR1, représente un radical bifonctionnel de formule 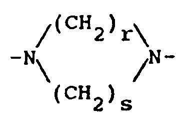 dans laquelle r et s sont l'un et l'autre 2;
et ses sels d'addition avec des acides pharmaceutiquement acceptables.

6. Composé selon la revendication 1, dans lequel
A représente un groupement N-[acyl(C₁₀-C₁₁) aliphatique]-β-D-2-désoxy-2-aminoglucopyrannosyle dans lequel le radical acyle aliphatique en C₁₀-C₁₁ est choisi parmi les radicaux (Z)-4-décénoyle, 8-méthylnonanoyle, décanoyle, 8-méthyldécanoyle et 9-méthyldécanoyle;
B est un atome d'hydrogène ou un groupe protecteur de la fonction amine;
M représente le radical a-D-mannopyrannosyle;
Y représente un groupe di- ou polyamino de formules:
-NH(CH₂)₃N(CH₃)₂;
-NH(CH₂)₃NH(CH₂)₂OH;
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂; 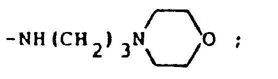
-NH(CH₂)₂NH(CH₂)₂NH₂;
-NH(CH₂)₃NH(CH_{z})₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂CH₂NH)₄CH₂CH₂NH_{z}; 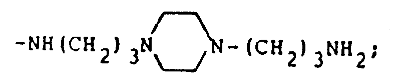 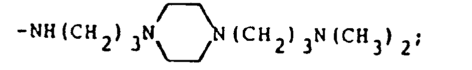 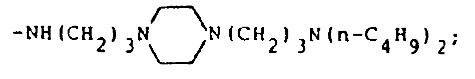
-N(CH₃)(CH₂)₃NHCH₃;
-N(CH₃)(CH₂)₃N(CH₃)₂; 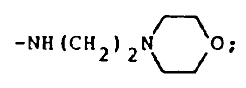
-NH(CH₂)₃N[(CH₂)3NH₂]₂
et ses sels d'addition avec des acides pharmaceutiquement acceptables.

7. Composé selon la revendication 6, dans lequel Y représente le groupe
-NH(CH₂)₃NH(CH₂)₄NH₂,
-NH(CH₂)₃NH(CH₂)₄NH(CH₂)₃NH₂ ou
-NH(CH₂)₃N[(CH₂)₃NH₂]₂
et ses sels d'addition avec des acides pharmaceutiquement acceptables.

8. Composé selon la revendication 6, dans lequel le radical acyle aliphatique est le radical 8-méthylnonanoyle ou
décanoyle, et Y représente le groupe
-NH(CH₂)₃NH(CH₂)₄NH₂,
-NH(CH₂)₃NH(CH₂)₄NH(CH₂)₃NH₂ ou
-NH(CH₂)₃N[(CH₂)₃NH₂]₂
et ses sels d'addition avec des acides pharmaceutiquement acceptables.

9. Procédé pour la préparation d'un dérivé de téicoplanine de la rivendication 1, comprenant l'amidation du produit carboxylique de départ, de type téicoplanine, de formule I, correspondant, dans lequel A, B, M ont les mêmes significations que dans la revendication 1, et Y est OH, avec une amine de formule II 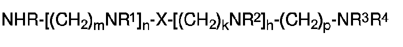 dans laquelle R, R1, R2, R3, R⁴, X, m, n, h, k et p ont les mêmes significations que dans la revendication 1 et, éventuellement, l'élimination du groupe protecteur de la fonction N'⁵⁻amino.

10. Procédé selon la revendication 9, dans lequel on effectue le processus d'amidation par conversion du produit carboxylique de départ en son ester activé correspondant, de préférence protégé sur la fonction N¹⁵-amino, et on fait réagir l'ester activé avec un excès molaire d'une amine de formule II, en présence d'un solvant organique polaire, à une température comprise entre 5 et 60°C, de préférence entre 10 et 30°C.

11. Substance telle que définie dans l'une quelconque des revendications 1 à 8, pour utilisation en tant que médicament.

12. Utilisation d'une substance telle que définie dans l'une quelconque des revendications 1 à 8, pour la fabrication d'un médicament destiné à combattre des infections provoquées par des streptocoques du groupe A et/ou des staphylocoques coagulase-négatifs.

13. Composition pharmaceutique contenant un composé de l'une quelconque des revendications 1 à 8, en tant que composant actif.

## Revendications (Revendications pour l'(les) Etat(s) contractant(s) suivant(s) : ES, GR)

1. Procédé pour la préparation d'un dérivé de téicoplanine de formule 1 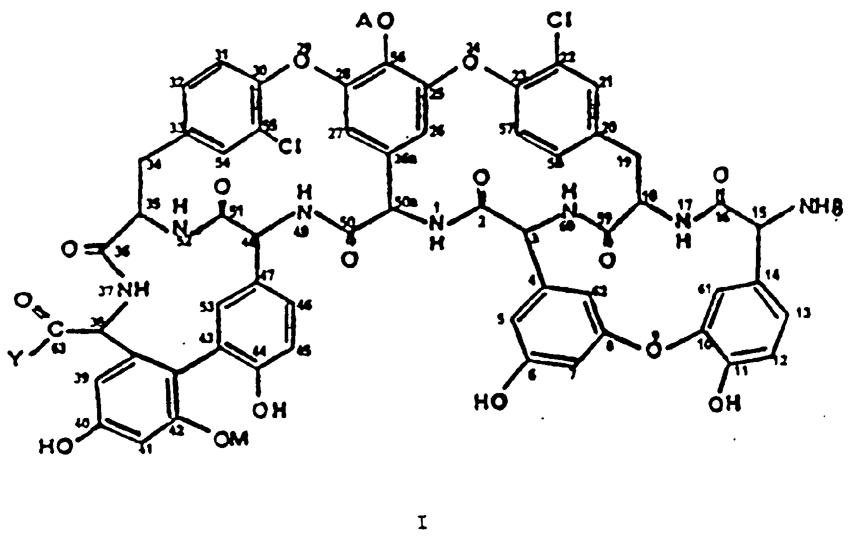 dans laquelle
A représente un groupememt N-[acyl(C₉-C₁₂) aliphatique]-β-β-D-2-désoxy-2-aminoglucopyrannosyle,
B est un atome d'hydrogène ou un groupe protecteur de la fonction amine;
M représente le groupe a-D-mannopyrannosyle,
Y représente un groupe di- ou polyamino de formule 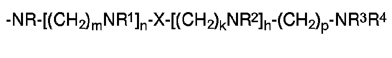 dans laquelle
R est un atome d'hydrogène ou un groupe alkyle en C₁-C₈ linéaire ou ramifié;
R¹ est un atome d'hydrogène ou un groupe alkyle en C₁-C₈ linéaire ou ramifié;
R² est un atome d'hydrogène ou un groupe alkyle en C₁-C₈ linéaire ou ramifié;
R3 et R⁴ sont, indépendamment l'un de l'autre, un atome d'hydrogène ou un groupe alkyle en C₁-C₈ linéaire ou ramifié, portant éventuellement un substituant NH₂, OH ou SH, ou pris ensemble, avec l'atome d'azote contigu, forment un cycle hétérocyclique saturé à 5-7 chaînons pouvant contenir un autre hétéroatome choisi parmi -S-, -O- et -NR⁵⁻, R⁵ étant un atome d'hydrogène ou un groupe alkyle en C₁-C₄, phényle ou phénylalkyle en Ci-C₄;
m, k et p représentent, indépendamment les uns des autres, un nombre entier allant de 2 à 8;
n et h représentent, indépendamment l'un de l'autre, un nombre entier allant de 0 à 4;
X représente une simple liaison, ou, lorsque n est 1, peut représenter, conjointement avec le groupe contigu NR1, un radical bifonctionnel de formule 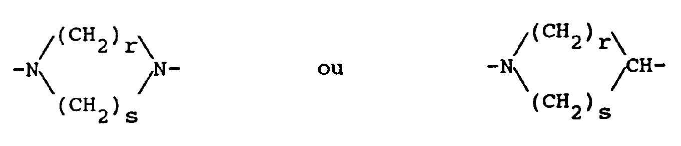 r et s représentant, indépendamment l'un de l'autre, un nombre entier allant de 1 à 6, étant entendu que leur somme est un nombre entier allant de 3 à 8;
et de ses sels d'addition avec des acides,
comprenant l'amidation du produit carboxylique de départ, de type téicoplanine, de formule I, correspondant, dans lequel A, B, M ont les mêmes significations que ci-dessus, et Y est OH, avec une amine de formule II 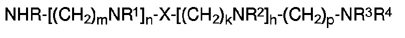 dans laquelle R, R1, R², R3, R⁴, X, m, n, h, k et p ont les mêmes significations que ci-dessus, et, éventuellement, l'élimination du groupe protecteur de la fonction N¹⁵⁻amino.

2. Procédé selon la revendication 1, dans lequel on effectue le processus d'amidation par conversion du produit carboxylique de départ en son ester activé correspondant, de préférence protégé sur la fonction N¹⁵-amino, et on fait réagir l'ester activé avec un excès molaire d'une amine de formule II, en présence d'un solvant organique polaire, à une température comprise entre 5 et 60°C, de préférence entre 10 et 30°C.

3. Procédé selon la revendication 2, dans lequel l'ester activé est l'ester cyanométhylique et sa proportion molaire par rapport à l'amine va de 1:10 à 1:20.

4. Procédé selon la revendication 3, dans lequel on prépare l'ester cyanométhylique en faisant réagir le produit de départ de type acide carboxylique, de préférence protégé sur la fonction N¹⁵⁻amino, avec un excès molaire d'environ 20-30 fois de chloracétonitrile, en présence d'un solvant organique inerte et d'une base qui n'interfère pas avec le cours de la réaction, à une température comprise entre 10 et 60°C, de préférence entre 15 et 30°C.

5. Procédé selon la revendication 1, dans lequel on effectue le processus d'amidation en faisant réagir le produit carboxylique de départ de type téicoplanine avec l'amine de formule Il dans un solvant organique inerte, en présence d'un agent de condensation choisi parmi un phosphorazidate d'alkyle en Ci-C₄, de phényle ou de radical hétérocyclique, à une température comprise entre 0 et 20°C.

6. Procédé selon l'une quelconque des revendications 1 à 4, pour la préparation de composés de formule 1 dans lesquels le radical acyle aliphatique en C₉-C₁₂ du fragment représenté par le symbole A est:
le groupe (Z)-4-décénoyle, 8-méthylnonanoyle, décanoyle, 8-méthyldécanoyle, 9-méthyldécanoyle, 6-méthylocta- noyle, nonanoyle, 10-méthylundécanoyle ou dodécanoyle; B est un atome d'hydrogène ou un groupe protecteur de la fonction amine, R, R¹ et R² sont des atomes d'hydrogène ou des radicaux alkyle linéaires ou ramifiés ayant de 1 à 4 atomes de carbone, R³ et R⁴ sont, indépendamment l'un de l'autre, un atome d'hydrogène ou un radical alkyle linéaire ou ramifié ayant de 1 à 4 atomes de carbone, portant éventuellement un substituant NH₂, OH ou SH, ou R3 et R⁴ pris ensemble, avec l'atome d'azote contigu, représentent l'un des cycles suivants:
pyrrolidine, pipéridine, oxazolidine, thiazolidine, isoxazolidine, isothiazolidine, morpholine, pipérazine, thiomorpholine, hexahydroazépine, hexahydro-1,5-diazépine et hexahydro-1,4-diazépine; R⁵ est un atome d'hydrogène ou un groupe alkyle en Cₗ-C₄;
les symboles m, k et p représentent des nombres entiers allant de 2 à 6, de préférence de 2 à 4;
les symboles n et h représentent 0, 1 ou 2, de préférence 0 ou 1;
le symbole X représente une simple liaison, ou, lorsque n est 1, pris ensemble avec le groupe contigu NR¹
représente un radical bifonctionnel de formule 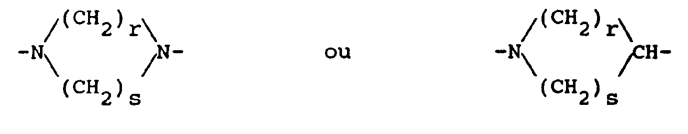
r et s étant l'un et l'autre 2 ou l'un étant 1 et l'autre étant 2 ou 3;
et de leurs sels d'addition avec des acides pharmaceutiquement acceptables.

7. Procédé selon l'une quelconque des revendications 1 à 4, pour la préparation d'un composé de formule 1 dans lequel le radical acyle aliphatique du fragment représenté par le symbole A est un radical acyle aliphatique en C₁₀-C₁₁ choisi parmi les radicaux (Z)-4-décénoyle, 8-méthylnonanoyle, décanoyle, 8-méthyldécanoyle et 9-méthyldécanoyle; B est un atome d'hydrogène ou un groupe protecteur de la fonction amine;
R est un atome d'hydrogène ou le groupe méthyle, R¹ et R² sont des atomes d'hydrogène, R³ et R⁴ sont, indépendamment l'un de l'autre, un atome d'hydrogène ou un groupe alkyle linéaire ou ramifié ayant de 1 à 4 atomes de carbone, portant éventuellement un substituant NH₂, OH ou SH, ou pris ensemble avec l'atome d'azote contigu, représentent un cycle pyrrolidine, morpholine ou pipérazine, et R⁵ est un atome d'hydrogène ou le groupe méthyle;
les symboles m, k et p représentent des nombres entiers allant de 2 à 4;
les symboles n et h représentent 0 ou 1 ;
le symbole X représente une simple liaison, ou, lorsque n est 1, pris ensemble avec le groupe contigu NR1, représente un radical bifonctionnel de formule 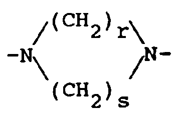
dans laquelle r et s sont l'un et l'autre 2;
et de ses sels d'addition avec des acides pharmaceutiquement acceptables.

8. Procédé selon l'une quelconque des revendications 1 à 4, pour la préparation d'un composé de formule I dans lequel
A représente un groupement N-[acyl(C₁₀-C₁₁) aliphatique]-β-D-2-désoxy-2-aminoglucopyrannosyle dans lequel le radical acyle aliphatique en C₁₀-C₁₁ est choisi parmi les radicaux (Z)-4-décénoyle, 8-méthylnonanoyle, décanoyle, 8-méthyldécanoyle et 9-méthyldécanoyle;
B est un atome d'hydrogène ou un groupe protecteur de la fonction amine;
M représente le radical a-D-mannopyrannosyle;
Y représente un groupe di- ou polyamino de formules:
-NH(CH₂)₃N(CH₃)₂;
-NH(CH₂)₃NH(CH₂)₂OH;
-NH(CH₂)₃NH(CH₂)₄NH₂;
-NH(CH₂)₄NH(CH₂)₃NH₂;
-NH(CH₂)₃NH(CH₂)₄NH(CH₂)₃NH₂;
-NH(CH₂)₂NH(CH₂)₂NH₂; 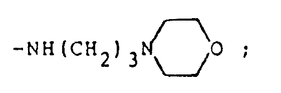
-NH(CH₂)₃NH(CHz)₃NH₂;
-NH(CH₂)₃NH(CH₂)₂NH(CH₂)₃NH₂;
-NH(CH₂)₃NH(CH₂)₃NH(CH₂)₃NH₂;
-NH(CH₂CH₂NH)₄CH₂CH₂NH₂; 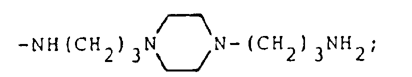 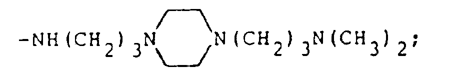 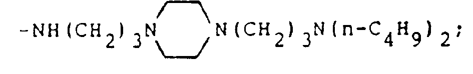
-N(CH₃)(CH₂)₃NHCH₃;
-N(CH₃)(CH₂)₃N(CH₃)₂; 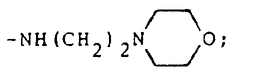
-NH(CH₂)₃N[(CH₂)₃NH₂]2
et de ses sels d'addition avec des acides pharmaceutiquement acceptables.

9. Procédé selon l'une quelconque des revendications 1 à 4, pour la préparation d'un composé de formule dans lequel A, B, et M ont le memes significations que dans la rivendication 8, et Y est -NH(CH₂)₃NH(CH₂)₄NH₂,-NH(CH₂)₃NH(CH₂)₄NH(CH₂)₃NH₂ ou -NH(CH₂)₃N[(CH₂)₃NH₂]₂ et de ses sels d'addition avec des acides pharmaceutiquement acceptables.

10. Procédé selon l'une quelconque des revendications 1 à 4, pour la préparation d'un composé de formule I dans lequel le radical acyle aliphatique du fragment représenté par le symbole A est le radical 8-méthylnonanoyle ou décanoyle, B est un atome dd'hydrogène ou un groupe protecteur de fonction amino, M représente le groupe a-D-mannopyrannosyle et Y représente le groupe
-NH(CH₂)₃NH(CH₂)₄NH₂,
-NH(CH₂)₃NH(CH₂)₄NH(CH₂)₃NH₂ ou
-NH(CH₂)₃N[(CH₂)₃NH₂]2
et de ses sels d'addition avec des acides pharmaceutiquement acceptables.
